# EUROPEAN PATENT APPLICATION

(11) **EP 4 495 600 A1**
(43) Date of publication of application: **22.01.2025**
(21) Application number: 24189667.9
(22) Date of filing: 19.07.2024
(51) Int. Cl.: G01N 35/00, C12N 15/10, G01N 33/543

(54) **DEVICES AND SYSTEMS FOR AUTOMATED ASSAYS AND USES THEREOF**

(30) Priority: 20.07.2023 US 202363514771 P
(71) Applicant: Plexbio Co., Ltd., Taipei City, 11491 (TW)
(72) Inventor: TSAO, Dean, Taipei City (TW); LU, Fengkan, Taipei City (TW); LIN, Cheng-Tse, Taipei City (TW)
(74) Representative: Müller Schupfner & Partner Patent- und Rechtsanwaltspartnerschaft mbB (Muc)

(57) **Abstract**

The present disclosure relates generally to devices and systems for performing automated multiplex assays, and methods of using such devices and systems. In some embodiments, the devices and systems comprise a magnetic module that simplifies and automates processing of encoded microcarriers for molecular and immunoassays.

## Description

### CROSS-REFERENCE TO RELATED APPLICATIONS

This application claims priority to U.S. Provisional Application Ser. No. 63/514,771, filed July 20, 2023, the contents of which is hereby incorporated by reference in their entirety.

### FIELD

The present disclosure relates generally to devices and systems for performing automated multiplex assays, and methods of using such devices and systems. In some embodiments, the devices and systems comprise a magnetic module that simplifies and automates processing of magnetic microcarriers for molecular and immunoassays.

### BACKGROUND

Immunological and molecular diagnostic assays play a critical role both in the research and clinical fields. Often it is necessary to perform assays for a panel of multiple targets to gain a meaningful or bird's-eye view of results to facilitate research or clinical decision-making. This is particularly true in the era of genomics and proteomics, where an abundance of genetic markers and/or biomarkers are thought to influence or be predictive of particular disease states. In theory, assays of multiple targets can be accomplished by testing each target separately in parallel or sequentially in different reaction vessels (i.e., multiple singleplexing). However, not only are assays adopting a singleplexing strategy often cumbersome, but they also typically required large sample volumes, especially when the targets to be analyzed are large in number.

A multiplex assay simultaneously measures multiple analytes (two or more) in a single assay. Multiplex assays are commonly used in high-throughput screening settings, where many specimens can be analyzed at once. It is the ability to assay many analytes simultaneously and many specimens in parallel that is the hallmark of multiplex assays and is the reason that such assays have become a powerful tool in fields ranging from drug discovery to functional genomics to clinical diagnostics. In contrast to singleplexing, by combining all targets in the same reaction vessel, the assay is much less cumbersome and much easier to perform, since only one reaction vessel is handled per sample. The required test samples can thus be dramatically reduced in volume, which is especially important when samples (e.g., tumor tissues, cerebral spinal fluid, or bone marrow) are difficult and/or invasive to retrieve in large quantities. Equally important is the fact that the reagent cost can be decreased and assay throughput increased drastically.

Existing systems for multiplex assays or for processing microcarriers generally require complicated and cumbersome structural elements such as suction channels, dispensing channels, a shaker table, and/or a magnetic plate. Such systems generally are challenging to maintain, for example, to avoid contamination or clogging (e.g., due to crystallization) in the suction and dispensing channels, and may take up too much space. Further, use of the shaker table may consume more energy and generate more noise compared to a more localized mixing method. Therefore, a need exists for an assay system that is compact, simple, and/or easy to maintain, e.g., one that does not require suction channels, dispensing channels, and/or a shaker table to process microcarriers.

### BRIEF SUMMARY

In one aspect, provided herein are magnetic modules for reacting or labeling magnetic microcarriers. In some embodiments, the module comprises one or more well sets. In some embodiments, each well set comprises a reaction well. In some embodiments, each well set comprises one or more reaction wash wells comprising a first reaction wash well and a last reaction wash well, each containing a reaction wash buffer. In some embodiments, the first reaction wash well and the last reaction wash well are the same well when there is only one reaction wash well in the well set. In some embodiments, each well set comprises a labeling well containing labeling molecules in a labeling buffer. In some embodiments, each well set comprises one or more labeling wash wells comprising a first labeling wash well and a last labeling wash well, each containing a labeling wash buffer. In some embodiments, the first labeling wash well and the last labeling wash well are the same well when there is only one labeling wash well in the well set. In some embodiments, the module comprises one or more magnetic units. In some embodiments, each magnetic unit is configured to move into, out of, and between each of the wells in a well set. In some embodiments, each magnetic unit is configured to attract the magnetic microcarriers and move the magnetic microcarriers into, out of, and between each of the wells in the well set. In some embodiments, each magnetic unit comprises an upper end and a lower end. In some embodiments, the module comprises one or more magnetic unit pockets. In some embodiments, each magnetic unit pocket is positioned between each magnetic unit and a well set. In some embodiments, each magnetic unit pocket is configured to move into, out of, and between each of the well in the well set. In some embodiments, each magnetic unit pocket is configured to at least partially cover the lower end of each magnetic unit when the magnetic unit moves or slides into the magnetic unit pocket. In some embodiments, each magnetic unit pocket comprises an interior surface and an exterior surface. In some embodiments, the magnetic microcarriers are coupled to one or more capture agents. In some embodiments, the one or more capture agents can bind to one or more analytes.

In some embodiments, each well set further comprises an antibody well containing secondary antibodies in an antibody buffer. In some embodiments, the secondary antibodies can bind to the one or more analytes and are coupled to one or more secondary binding moieties. In some embodiments, each well set further comprises one or more antibody wash wells, comprising a first antibody wash well and a last antibody wash well, each containing an antibody wash buffer. In some embodiments, the first antibody wash well and the last antibody wash well are the same well when there is only one antibody wash well in the well set.

In some embodiments, the reaction well, one or more reaction wash wells, antibody well, one or more antibody wash wells, labeling well, and one or more labeling wash wells are positioned along a straight or curved line in such order. In some embodiments, the secondary antibodies are selected from the group consisting of antibodies and antibody fragments. In some embodiments, the one or more secondary binding moieties can bind to labeling molecules. In some embodiments, the one or more secondary binding moieties comprise biotin.

In some embodiments, the reaction well, one or more reaction wash wells, labeling well, and one or more labeling wash wells are positioned along a straight or curved line in such order. In some embodiments, the one or more capture agents are each independently selected from the group consisting of small molecules, polynucleotides, polypeptides, proteins, lipids, and polysaccharides. In some embodiments, the one or more analytes are each independently selected from the group consisting of small molecules, polynucleotides, polypeptides, proteins, lipids, and polysaccharides.

In some embodiments, the one or more capture agents and the one or more analytes are each independently selected from the group consisting of polynucleotides. In some embodiments, the one or more capture agents are each independently selected from the group consisting of antibodies and antibody fragments, and the one or more analytes are each independently selected from the group consisting of antigens. In some embodiments, the one or more capture agents are each independently selected from the group consisting of antigens, and the one or more analytes are each independently selected from the group consisting of antibodies.

In some embodiments, the labeling molecules comprise a label binding moiety coupled to a reporting moiety. In some embodiments, the label binding moiety is streptavidin (SA) or anti-biotin antibody. In some embodiments, the reporting moiety is phycoerythrin (PE) or fluorescein isothiocyanate (FITC).

In some embodiments, each magnetic unit has a rod-like shape. In some embodiments, each magnetic unit pocket has a closed-bottom cylindrical shape. In some embodiments, the one or more magnetic unit pockets are configured to move or oscillate vertically relative to the one or more well sets. In some embodiments, the one or more magnetic unit pockets are disposable. In some embodiments, the number of the one or more magnetic units and the number of the one or more magnetic unit pockets are the same. In some embodiments, the number of the one or more magnetic units and the number of reaction wells are the same.

In some embodiments, each type of wells is positioned along a line or forms a one-dimensional arrangement. In some embodiments, the reaction wells are positioned along a line, or wherein the labeling wells are positioned along a line, or both. In some embodiments, the one or more well sets form a multi-well plate, wherein each well set forms each row of the multi-well plate, and wherein each type of wells forms each column of the multi-well plate. In some embodiments, the one or more magnetic units are positioned along a straight line above the multi-well plate and parallel to the column direction of the multi-well plate. In some embodiments, the one or more magnetic unit pockets are positioned along a straight line between the multi-well plate and the one or more magnetic units and parallel to the column direction of the multi-well plate. In some embodiments, each type of wells is positioned along a surface or forms a two-dimensional arrangement. In some embodiments, the reaction wells are positioned along a surface or form a two-dimensional arrangement, or wherein the labeling wells are positioned along a surface or form a two-dimensional arrangement, or both. In some embodiments, each type of wells forms a multi-well plate, such that the one or more well sets comprise a plurality of multi-well plates. In some embodiments, the one or more magnetic units and each type of wells are in the same one- or two-dimensional arrangement such that the one or more magnetic units can simultaneously move into and out of each type of wells. In some embodiments, the one or more magnetic unit pockets and each type of wells are in the same one- or two-dimensional arrangement such that the one or more magnetic unit pockets can simultaneously move into and out of each type of wells.

In some embodiments, the reaction well contains the encoded microcarriers or a reaction buffer, or both. In some embodiments, the magnetic microcarriers comprise encoded microcarriers, spherical magnetic beads, or a combination thereof. In some embodiments, the encoded microcarriers each comprises: a substantially transparent magnetic polymer layer having a first surface and a second surface, the first and the second surfaces being parallel to each other, wherein the substantially transparent magnetic polymer comprises a mixture of a substantially transparent polymer and a plurality of magnetic nanoparticles, and wherein the magnetic nanoparticles comprise iron(II,III) oxide or iron(III) oxide. In some embodiments, the encoded microcarriers each comprises a substantially non-transparent layer, wherein the substantially non-transparent layer is affixed to the first surface of the substantially transparent magnetic polymer layer, and wherein an outline of the substantially non-transparent layer constitutes a two-dimensional shape representing an analog code. In some embodiments, the encoded microcarriers each comprises one or more capture agents for capturing one or more analytes, wherein the one or more capture agents are coupled to at least one of the first surface and the second surface of the substantially transparent magnetic polymer layer. In some embodiments, the magnetic component of the encoded microcarriers comprise the substantially transparent magnetic polymer layer. In some embodiments, the magnetic nanoparticles are superparamagnetic. In some embodiments, the magnetic nanoparticles are less than about 30 nm in diameter and greater than or equal to about 3 nm in diameter. In some embodiments, the plurality of magnetic nanoparticles comprises less than about 10% (by weight) and greater than about 0.1% (by weight) of the mixture of the substantially transparent polymer and the plurality of magnetic nanoparticles. In some embodiments, the substantially transparent magnetic polymer layer is between about 0.1 µm and about 50 µm in thickness. In some embodiments, the substantially transparent polymer is an epoxy-based polymer. In some embodiments, the epoxy-based polymer is SU-8. In some embodiments, the substantially non-transparent layer comprises a substantially non-transparent polymer. In some embodiments, the substantially non-transparent layer comprises a black matrix resist. In some embodiments, the substantially non-transparent polymer exhibits an absorbance of greater than about 1.8 (OD) at a wavelength between about 230 nm and about 660 nm. In some embodiments, the substantially non-transparent layer comprises a metal that lacks residual magnetism. In some embodiments, the substantially non-transparent layer comprises titanium or chromium. In some embodiments, the substantially non-transparent layer is between about 0.05 µm and about 2 µm in thickness. In some embodiments, the analog code comprises one or more overlapping or partially overlapping arc elements forming a continuous or discontinuous ring. In some embodiments, encoded microcarriers each comprises an orientation indicator for orienting the analog code of the substantially non-transparent layer. In some embodiments, the orientation indicator comprises an asymmetry of the substantially non-transparent layer. In some embodiments, the microcarrier is a substantially circular disc. In some embodiments, the microcarrier is between about 5 µm and about 200 µm in diameter, or wherein the microcarrier is about 40 µm in diameter. In some embodiments, the microcarrier is less than about 50 µm in thickness, or wherein the microcarrier is between about 2 µm and about 10 µm in thickness, or wherein the microcarrier is about 5 µm in thickness. In some embodiments, the one or more analytes and the one or more capture agents are each independently selected from the group consisting of a DNA molecule, a DNA-analog-molecule, an RNA-molecule, an RNA-analog-molecule, a polynucleotide, a protein, an enzyme, a lipid, a phospholipid, a carbohydrate moiety, a polysaccharide, an antigen, a virus, a cell, an antibody, a small molecule, a bacterial cell, a cellular organelle, and an antibody fragment.

In some aspect, provided herein are assay systems comprising the magnetic modules described herein. In some embodiments, the system further comprises a detection module configured to detect signal from the magnetic microcarriers. In some embodiments, the detection module comprises one or more detection wells. In some embodiments, the one or more detection wells have a flat bottom. In some embodiments, the one or more detection wells comprise the last labeling wash well. In some embodiments, the one or more detection wells and the one or more magnetic units have the same one- or two-dimensional arrangement such that the one or more magnetic units can simultaneously move into and out of the one or more detection wells. In some embodiments, the one or more detection wells and the one or more magnetic unit pockets have the same one- or two-dimensional arrangement such that the one or more magnetic unit pockets can simultaneously move into and out of the one or more detection wells.

In some embodiments, the assay system further comprises a PCR module configured to perform polymerase chain reaction (PCR). In some embodiments, the PCR module is configured to perform PCR using primers coupled to one or more secondary binding moieties that can bind to the labeling molecules. In some embodiments, the secondary binding moieties comprise biotin.

In some embodiments, the assay system further comprises a liquid handling module. In some embodiments, the liquid handling module is configured to transfer sample from the PCR module to the magnetic module. In some embodiments, the liquid handling module is configured to transfer sample from the PCR module to the reaction well of the magnetic module.

In some embodiments, each well set further comprises a hybridization buffer well containing a hybridization buffer, and the liquid handling module is configured to transfer at least part of the hybridization buffer from the hybridization buffer well to the reaction well. In some embodiments, each well set further comprises an incubation buffer well containing an incubation buffer, and the liquid handling module is configured to transfer at least part of the incubation buffer from the incubation buffer well to the reaction well.

In some aspects, provided herein are methods of using the magnetic module or the assay system described herein. In some embodiments, the method comprises (a) transferring one or more analytes into the reaction well, wherein the reaction well contains the magnetic microcarriers in a reaction buffer. In some embodiments, the method comprises (b) moving one of the one or more magnetic unit pockets into the reaction well and moving or oscillating the magnetic unit pocket in the reaction well to mix the one or more analytes and the magnetic microcarriers. In some embodiments, the method comprises (c) moving the magnetic unit into the reaction well and into the magnetic unit pocket to attract the magnetic microcarriers to the exterior surface of the magnetic unit pocket. In some embodiments, the method comprises (d) moving the magnetic unit pocket and magnetic unit out of the reaction well and into the first reaction wash well to transfer the magnetic microcarriers from the reaction well to the first reaction wash well. In some embodiments, the method comprises (e) moving the magnetic unit pocket and magnetic unit out of the last reaction wash well and into the labeling well to transfer the magnetic microcarriers from the last reaction wash well to the labeling well. In some embodiments, the method comprises (f) moving the magnetic unit out of the labeling well while keeping the magnetic unit pocket in the labeling well, thereby releasing the magnetic microcarriers from the exterior surface of the magnetic unit pocket into the labeling buffer. In some embodiments, the method comprises (g) moving the magnetic unit into the labeling well and into the magnetic unit pocket to attract the magnetic microcarriers to the exterior surface of the magnetic unit pocket. In some embodiments, the method comprises (h) moving the magnetic unit pocket and magnetic unit out of the labeling well and into the first labeling wash well to transfer the magnetic microcarriers from the labeling well to the first labeling wash well. In some embodiments, steps (a) to (h) occur in the order listed. In some embodiments, the method further comprises, after step (d) and before step (e), moving the magnetic unit out of the first reaction wash well while keeping the magnetic unit pocket in the first reaction wash well, thereby releasing the magnetic microcarriers from the exterior surface of the magnetic unit pocket into the first reaction wash well. In some embodiments, the method further comprises, after step (d) and before step (e), moving or oscillating the magnetic unit pocket in the first reaction wash well to mix the magnetic microcarriers with the reaction wash buffer. In some embodiments, the method further comprises, after step (f) and before step (g), moving or oscillating the magnetic unit pocket in the labeling well to mix the magnetic microcarriers with the labeling buffer. In some embodiments, the method further comprises, after step (h), moving the magnetic unit out of the first labeling wash well while keeping the magnetic unit pocket in the first labeling wash well, thereby releasing the magnetic microcarriers from the exterior surface of the magnetic unit pocket into the first labeling wash well. In some embodiments, the method further comprises, after step (h), moving or oscillating the magnetic unit pocket in the first labeling wash well to mix the magnetic microcarriers with the labeling wash buffer. In some embodiments, the method further comprises, after step (h), moving the magnetic unit pocket and magnetic unit out of the last labeling wash well and into a detection well to transfer the magnetic microcarriers from the last reaction wash well to the detection well. In some embodiments, the method further comprises moving the magnetic unit out of the detection well while keeping the magnetic unit pocket in the detection well, thereby releasing the magnetic microcarriers from the exterior surface of the magnetic unit pocket into the detection well. In some embodiments, the method further comprises, after step (h), optically reading the magnetic microcarriers and detecting signal from labeling molecules coupled to the magnetic microcarriers.

In some embodiments, the method comprises (a) transferring one or more analytes into the reaction well, wherein the reaction well contains the magnetic microcarriers in a reaction buffer. In some embodiments, the method comprises (b) moving one of the one or more magnetic unit pockets into the reaction well and moving or oscillating the magnetic unit pocket in the reaction well to mix the one or more analytes and the magnetic microcarriers. In some embodiments, the method comprises (c) moving the magnetic unit into the reaction well and into the magnetic unit pocket to attract the magnetic microcarriers to the exterior surface of the magnetic unit pocket. In some embodiments, the method comprises (d) moving the magnetic unit pocket and magnetic unit out of the reaction well and into the first reaction wash well to transfer the magnetic microcarriers from the reaction well to the first reaction wash well. In some embodiments, the method comprises (e) moving the magnetic unit pocket and magnetic unit out of the last reaction wash well and into the antibody well to transfer the magnetic microcarriers from the last reaction wash well to the antibody well. In some embodiments, the method comprises (f) moving the magnetic unit out of the antibody well while keeping the magnetic unit pocket in the labeling well, thereby releasing the magnetic microcarriers from the exterior surface of the magnetic unit pocket into the antibody buffer. In some embodiments, the method comprises (g) moving the magnetic unit into the antibody well and into the magnetic unit pocket to attract the magnetic microcarriers to the exterior surface of the magnetic unit pocket. In some embodiments, the method comprises (h) moving the magnetic unit pocket and magnetic unit out of the antibody well and into the first antibody wash well to transfer the magnetic microcarriers from the antibody well to the first antibody wash well. In some embodiments, the method comprises (i) moving the magnetic unit pocket and magnetic unit out of the last antibody wash well and into the labeling well to transfer the magnetic microcarriers from the last antibody wash well to the labeling well. In some embodiments, the method comprises (j) moving the magnetic unit out of the labeling well while keeping the magnetic unit pocket in the labeling well, thereby releasing the magnetic microcarriers from the exterior surface of the magnetic unit pocket into the labeling buffer. In some embodiments, the method comprises (k) moving the magnetic unit into the labeling well and into the magnetic unit pocket to attract the magnetic microcarriers to the exterior surface of the magnetic unit pocket. In some embodiments, the method comprises (l) moving the magnetic unit pocket and magnetic unit out of the labeling well and into the first labeling wash well to transfer the magnetic microcarriers from the labeling well to the first labeling wash well. In some embodiments, steps (a) to (1) occur in the order listed. In some embodiments, the method comprises, after step (d) and before step (e), moving the magnetic unit out of the first reaction wash well while keeping the magnetic unit pocket in the first reaction wash well, thereby releasing the magnetic microcarriers from the exterior surface of the magnetic unit pocket into the first reaction wash well. In some embodiments, the method comprises, after step (d) and before step (e) moving or oscillating the magnetic unit pocket in the first reaction wash well to mix the magnetic microcarriers with the reaction wash buffer. In some embodiments, the method comprises, after step (f) and before step (g), moving or oscillating the magnetic unit pocket in the antibody well to mix the magnetic microcarriers with the labeling buffer. In some embodiments, the method comprises, after step (h) and before step (i) moving the magnetic unit out of the first antibody wash well while keeping the magnetic unit pocket in the first antibody wash well, thereby releasing the magnetic microcarriers from the exterior surface of the magnetic unit pocket into the first antibody wash well. In some embodiments, the method comprises, after step (h) and before step (i) moving or oscillating the magnetic unit pocket in the first antibody wash well to mix the magnetic microcarriers with the antibody wash buffer. In some embodiments, the method comprises, after step (j) and before step (k), moving or oscillating the magnetic unit pocket in the labeling well to mix the magnetic microcarriers with the labeling buffer. In some embodiments, the method comprises, after step (1) moving the magnetic unit out of the first labeling wash well while keeping the magnetic unit pocket in the first labeling wash well, thereby releasing the magnetic microcarriers from the exterior surface of the magnetic unit pocket into the first labeling wash well. In some embodiments, the method comprises, after step (1) moving or oscillating the magnetic unit pocket in the first labeling wash well to mix the magnetic microcarriers with the labeling wash buffer. In some embodiments, the method comprises, after step (l), moving the magnetic unit pocket and magnetic unit out of the last labeling wash well and into a detection well to transfer the magnetic microcarriers from the last reaction wash well to the detection well. In some embodiments, the method comprises moving the magnetic unit out of the detection well while keeping the magnetic unit pocket in the detection well, thereby releasing the magnetic microcarriers from the exterior surface of the magnetic unit pocket into the detection well. In some embodiments, the method comprises, after step (l), optically reading the magnetic microcarriers and detecting signal from labeling molecules coupled to the magnetic microcarriers.

### DESCRIPTION OF THE FIGURES

The present application can be understood by reference to the following description taken in conjunction with the accompanying figures.
**FIG. 1** depicts an exemplary magnetic module.
**FIG. 2** depicts an exemplary assay system.
**FIG. 3** shows exemplary well sets where each type of wells forms a column of a multi-well plate.
**FIGS. 4A-4F** show exemplary well sets where each type of wells form a multi-well plate.
**FIG. 5** shows an exemplary process for a molecular assay using the magnetic modules provided herein.
**FIG. 6** shows an exemplary design of the well set for a molecular assay using the magnetic modules provided herein.
**FIG. 7** shows an exemplary process for an antigen immunoassay using the magnetic modules provided herein.
**FIG. 8** shows an exemplary design of the well set for an immunoassay using the magnetic modules provided herein (for either antigen or antibody detection).
**FIG. 9** shows an exemplary process for an antibody immunoassay using the magnetic modules provided herein.
**FIG. 10** shows the results of an antigen immunoassay performed with the magnetic module provided herein.

### DETAILED DESCRIPTION

The following description is presented to enable a person of ordinary skill in the art to make and use the various embodiments. Descriptions of specific devices, techniques, and applications are provided only as examples. Various modifications to the examples described herein will be readily apparent to those of ordinary skill in the art, and the general principles defined herein may be applied to other examples and applications without departing from the spirit and scope of the various embodiments. Thus, the various embodiments are not intended to be limited to the examples described herein and shown but are to be accorded the scope consistent with the claims.

### I. Definitions

Before describing the invention in detail, it is to be understood that this invention is not limited to particular compositions or biological systems, which can, of course, vary. It is also to be understood that the terminology used herein is for the purpose of describing particular embodiments only, and is not intended to be limiting.

The term "microcarrier" as used herein may refer to a physical substrate onto which a capture agent may be coupled. A microcarrier of the present disclosure may take any suitable geometric form or shape. In some embodiments, the microcarrier may be disc-shaped. Typically, the form or shape of a microcarrier will comprise at least one dimension on the order of 10⁻⁴ to 10⁻⁷ m (hence the prefix "micro").

The term "polymer" as used herein may refer to any macromolecular structure comprising repeated monomers. A polymer may be natural (e.g., found in nature) or synthetic (e.g., man-made, such as a polymer composed of non-natural monomer(s) and/or polymerized in a configuration or combination not found in nature). The terms "substantially transparent" and "substantially non-transparent" as used herein may refer to the ability of light (e.g., of a particular wavelength, such as infrared, visible, UV, and so forth) to pass through a substrate, such as a polymer layer. A substantially transparent polymer may refer to one that is transparent, translucent, and/or pervious to light, whereas a substantially non-transparent polymer may refer to one that reflects and/or absorbs light. It is to be appreciated that whether a material is substantially transparent or substantially non-transparent may depend upon the wavelength and/or intensity of light illuminating the material, as well as the means detecting the light traveling through the material (or a decrease or absence thereof). In some embodiments, a substantially non-transparent material causes a perceptible decrease in transmitted light as compared to the surrounding material or image field, e.g., as imaged by light microscopy (e.g., bright field, dark field, phase contrast, differential interference contrast (DIC), Nomarski interference contrast (NIC), Nomarski, Hoffman modulation contrast (HMC), or fluorescence microscopy). In some embodiments, a substantially transparent material allows a perceptible amount of transmitted light to pass through the material, e.g., as imaged by light microscopy (e.g., bright field, dark field, phase contrast, differential interference contrast (DIC), Nomarski interference contrast (NIC), Nomarski, Hoffman modulation contrast (HMC), or fluorescence microscopy).

The term "analog code" as used herein may refer to any code in which the encoded information is represented in a non-quantized and/or non-discrete manner, e.g., as opposed to a digital code. For example, a digital code is sampled at discrete positions for a limited set of values (e.g., 0/1 type values), whereas an analog code may be sampled at a greater range of positions (or as a continuous whole) and/or may contain a wider set of values (e.g., shapes). In some embodiments, an analog code may be read or decoded using one or more analog shape recognition techniques.

The term "capture agent" as used herein is a broad term and is used in its ordinary sense to refer to any compound or substance capable of specifically recognizing an analyte of interest. In some embodiments, specific recognition may refer to specific binding. Non-limiting examples of capture agents comprise, for example, a DNA molecule, a DNA-analog-molecule, an RNA-molecule, an RNA-analog-molecule, a polynucleotide, a protein, an enzyme, a lipid, a phospholipid, a carbohydrate moiety, a polysaccharide, an antigen, a virus, a cell, an antibody, a small molecule, a bacterial cell, a cellular organelle, and an antibody fragment.

"Analyte," as used herein, is a broad term and is used in its ordinary sense as a substance the presence, absence, or quantity of which is to be determined, comprising, without limitation, to refer to a substance or chemical constituent in a sample such as a biological sample or cell or population of cells that can be analyzed. An analyte can be a substance for which a naturally occurring binding member exists, or for which a binding member can be prepared. Non-limiting examples of analytes comprise, for example, antibodies, antibody fragments, antigens, polynucleotides (such as a DNA molecule, DNA-analog-molecule, RNA molecule, or RNA-analog-molecule), polypeptides, proteins, enzymes, lipids, phospholipids, carbohydrate moieties, polysaccharides, small molecules, organelles, hormones, cytokines, growth factors, steroids, vitamins, toxins, drugs, and metabolites of the above substances, as well as cells, bacteria, viruses, fungi, algae, fungal spores and the like.

The term "antibody" is used in the broadest sense and comprises monoclonal antibodies (comprising full length antibodies which have an immunoglobulin Fc region), polyclonal antibodies, multispecific antibodies (e.g., bispecific antibodies, diabodies, and single-chain molecules), as well as antibody fragments (e.g., Fab, F(ab')2, and Fv).

As used herein, "sample" refers to a composition containing a material, such as a molecule, to be detected. In one embodiment, the sample is a "biological sample" (i.e., any material obtained from a living source (e.g., human, animal, plant, bacteria, fungi, protist, virus)). The biological sample can be in any form, comprising solid materials (e.g., tissue, cell pellets and biopsies) and biological fluids (e.g., urine, blood, saliva, lymph, tears, sweat, prostatic fluid, seminal fluid, semen, bile, mucus, amniotic fluid and mouth wash (containing buccal cells)). Solid materials typically are mixed with a fluid. Sample can also refer to an environmental sample such as water, air, soil, or any other environmental source.

As used in this specification and the appended claims, the singular forms "a", "an" and "the" comprise plural referents unless the content clearly dictates otherwise. Thus, for example, reference to "a molecule" optionally comprises a combination of two or more such molecules, and the like.

The term "about" as used herein refers to the usual error range for the respective value readily known to the skilled person in this technical field. Reference to "about" a value or parameter herein comprises (and describes) embodiments that are directed to that value or parameter per se.

It is understood that aspects and embodiments of the invention described herein comprise "comprising," "consisting," and "consisting essentially of" aspects and embodiments.

### II. Magnetic Modules

In one aspect, described herein are magnetic modules. In some embodiments, the magnetic module is for reacting or labeling magnetic microcarriers. In some embodiments, the magnetic module comprises one or more well sets, one or more magnetic units, one or more magnetic unit pockets, or any combination thereof. In some embodiments, the magnetic microcarriers comprise a magnetic component. In some embodiments, the magnetic microcarriers are coupled to one or more capture agents, wherein the one or more capture agents can bind to one or more analytes.

In some embodiments, the one or more well sets comprise a reaction well. In some embodiments, the reaction well is configured to comprise a reaction buffer. Any suitable reaction buffers can be used. In some embodiments, the reaction buffer is a hybridization buffer. In some embodiments, the reaction buffer is an incubation buffer. In some embodiments, the reaction well is configured to comprise one or more magnetic microcarriers. In some embodiments, the reaction well is an empty well. In some embodiments, the reaction well is configured to comprise both a reaction buffer and magnetic microcarriers. In some embodiments, the magnetic microcarriers comprise encoded microcarriers described herein. In some embodiments, the magnetic microcarriers comprise magnetic latex beads. In some embodiments, the magnetic microcarriers comprise spherical magnetic beads. In some embodiments, the magnetic microcarriers comprise spherical magnetic latex beads.

In some embodiments, the one or more well sets comprise one or more reaction wash wells. In some embodiments, the one or more reaction wash wells comprise a first reaction wash well and a last reaction wash well, each configured to contain a reaction wash buffer. In some embodiments, there is only one reaction wash well in the well set, wherein the first reaction wash well and the last reaction wash well are the same well.

In some embodiments, the one or more well sets comprise a labeling well. In some embodiments, the labeling well contains labeling molecules in a labeling buffer. In some embodiments, the labeling molecule is a streptavidin-phycoerythrin (PE) conjugate.

In some embodiments, the one or more well sets comprise one or more labeling wash wells. In some embodiments, the one or more labeling wash wells comprise a first labeling wash well and a last labeling wash well, each configured to contain a labeling wash buffer. In some embodiments, there is only one labeling wash well in the well set, wherein the first labeling wash well and the last labeling wash well are the same well.

In some variations, the one or more well sets further comprise wells containing additional reagents, such as an antibody well and one or more antibody wash wells.

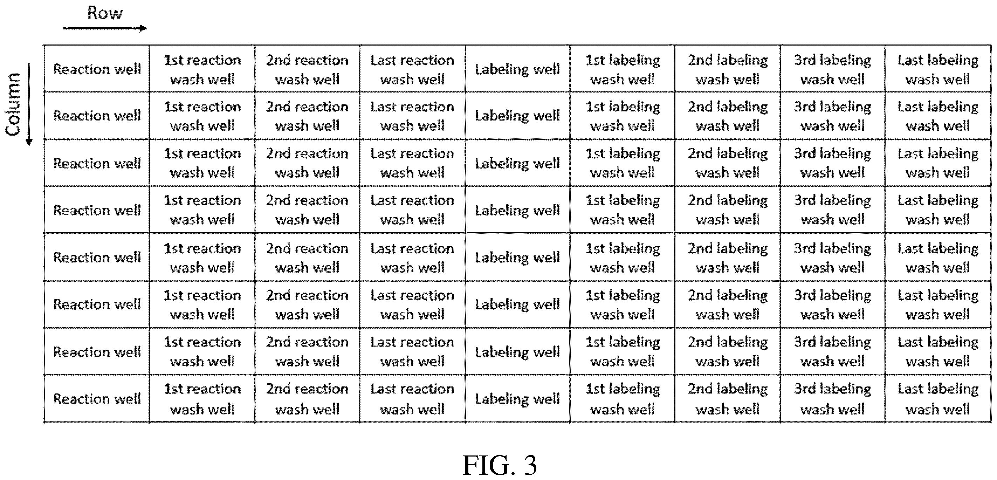

In some embodiments, each type of wells in the one or more well sets forms a one-dimensional arrangement. The one-dimensional arrangement may be a straight line, a set of straight lines connected with one another at the ends, or a curved line. FIG. 3 demonstrates an exemplary 8×9 arrangement of wells. A set of wells (a well set) comprising one of each type of wells (i.e., a reaction well, a first reaction wash well, a second reaction wash well, a last reaction wash well, a labeling well, a first labeling wash well, a second labeling wash well, a third labeling wash well, and a last labeling wash well) forms a row of this matrix arrangement. In some embodiments, the reaction wells are positioned along a line (e.g., FIG. 3). In some embodiments, the labeling wells are positioned along a line (e.g., FIG. 3). In some embodiments, the one or more well sets form a multi-well plate (e.g., FIG. 3). In FIG. 3, each type of wells (i.e., the reaction wells, the first reaction wash wells, the second reaction wash wells, the last reaction wash wells, the labeling wells, the first labeling wash wells, the second labeling wash wells, the third labeling wash wells, or the last labeling wash wells) forms each column of the 8×9 multi-well plate, and each row comprising one of each type of well forms a well set. In some embodiments, each well set forms each row of the multi-well plate (e.g., FIG. 3), and each type of wells forms each column of the multi-well plate (e.g., FIG. 3).

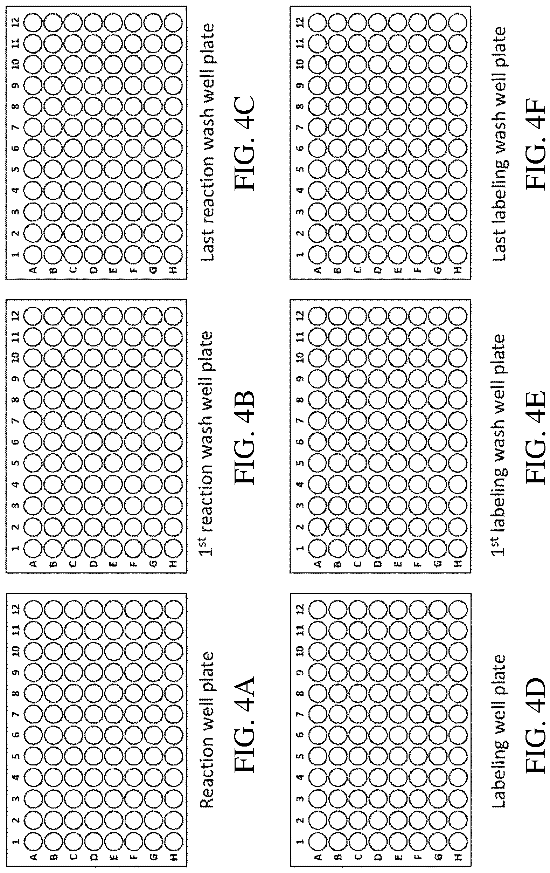

In some embodiments, each type of wells forms a two-dimensional arrangement. The two-dimensional arrangement may be along a surface (such as a multi-well plate). In some embodiments, the two-dimensional arrangement is a two-dimensional array. The array may take any shape, such as a square, a rectangle, a triangle, a circle, or a hexagon. In some embodiments, each type of wells forms a multi-well plate. In some embodiments, the one or more well sets comprise a plurality of multi-well plates. FIGS. 4A-4F show exemplary well sets comprising two-dimensional arrangement in the form of multi-well plates. FIG. 4A depicts a multi-well plate where the reaction wells are positioned along a flat surface to form an 8×12 two-dimensional arrangement. FIG. 4B depicts a multi-well plate where the first reaction wash wells are positioned along a flat surface to form an 8×12 two-dimensional arrangement. FIG. 4C depicts a multi-well plate where the last reaction wash wells are positioned along a flat surface to form an 8×12 two-dimensional arrangement. FIG. 4D depicts a multi-well plate where the labeling wells are positioned along a flat surface to form an 8×12 two-dimensional arrangement. FIG. 4E depicts a multi-well plate where the first labeling wash wells are positioned along a flat surface to form an 8×12 two-dimensional arrangement. FIG. 4F depicts a multi-well plate where the last labeling wash wells are positioned along a flat surface to form an 8×12 two-dimensional arrangement.

In some embodiments, the magnetic module comprises one or more magnetic units comprising an upper end and a lower end. In some embodiments, each magnetic unit is configured to move into, out of, and between each of the wells in a well set. In some embodiments, the magnetic module comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, or more magnetic units. In some embodiments, the magnetic module comprises 6, 12, 24, 48, 96, 192, or 384 magnetic units. In some embodiments, the magnetic module comprises 8 magnetic units. In some embodiments, the magnetic module comprises 96 magnetic units. In some embodiments, the magnetic module is configured to attract the magnetic microcarriers.

In some embodiments, the magnetic module comprises one or more magnetic unit pockets comprising an interior surface and an exterior surface. In some embodiments, the magnetic module can comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, or more magnetic unit pockets. In some embodiments, the magnetic module comprises 6, 12, 24, 48, 96, 192, or 384 magnetic unit pockets. In some embodiments, the magnetic module comprises 8 magnetic unit pockets. In some embodiments, the magnetic module comprises 96 magnetic unit pockets.

In some embodiments, the one or more magnetic units, the one or more magnetic unit pockets, and each type of wells are in the same one- or two-dimensional arrangement. For example, magnetic units, magnetic unit pockets, and labeling wells are in a 8×1 arrangement (i.e., 8 magnetic units form a line, 8 magnetic unit pockets for a line, and 8 labeling wells form a line). In some embodiments, magnetic units, magnetic unit pockets, and labeling wells are in a 8×12 arrangement (i.e., 96 magnetic units form an 8×12 arrangement, 96 magnetic unit pockets form an 8×12 arrangement, and 96 labeling wells form a 8×12 arrangement). In some embodiments, the one or more magnetic units and the one or more magnetic unit pockets are in the same one- or two-dimensional arrangement. In some embodiments, the one or more magnetic units and each type of wells are in the same one- or two-dimensional arrangement. In some embodiments, the one or more magnetic unit pockets and each type of wells are in the same one- or two-dimensional arrangement.

In some embodiments, the magnetic units and magnetic unit pockets can simultaneously move into and out of each type of wells. In some embodiments, the magnetic units can simultaneously move into and out of each type of wells. In some embodiments, magnetic unit pockets can simultaneously move into and out of each type of wells. In some embodiments, the magnetic units can simultaneously move into and out of each type of magnetic unit pockets.

In some embodiments, the magnetic unit pockets are positioned between each magnetic unit and a well set. In some embodiments, the magnetic unit pocket is configured to at least partially cover the lower end of each magnetic unit when the magnetic unit moves or slides into the magnetic unit pocket. In some embodiments, the number of the one or more magnetic units and the number of the one or more magnetic unit pockets are the same. In some embodiments, the number of the one or more magnetic units and the number of each type of wells are the same. In some embodiments, the number of the one or more magnetic units and the number of reaction wells are the same. In some embodiments, the number of the one or more magnetic units and the number of labeling wells are the same. In some embodiments, the one or more magnetic units are positioned along a straight light above the multi-well plate. In some embodiments, the one or more magnetic units are positioned parallel to the column direction of the multi-well plate, such as a multi-well plate shown in FIG. 3.

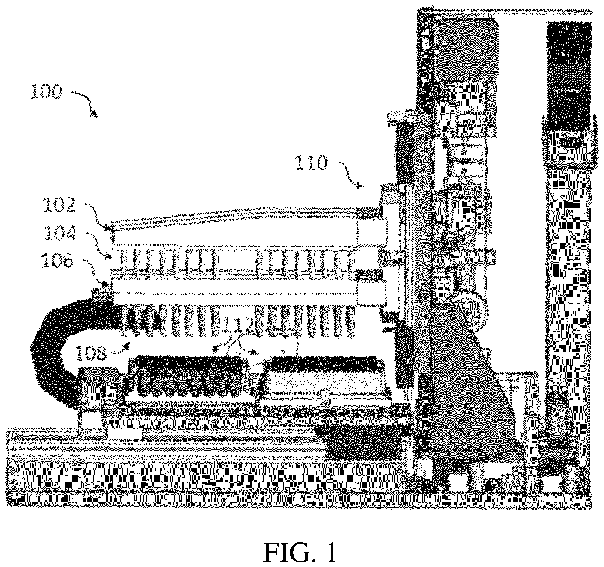

FIG. 1 shows exemplary magnetic module 100, comprising magnetic unit gantry 102, magnetic units 104, pocket gantry 106, magnetic unit pockets 108, position control unit 110, and well area 112. Magnetic units are attached to magnetic unit gantry, whose vertical and horizontal positions are controlled by position control unit. Magnetic unit pockets are connected to pocket gantry, whose vertical and horizontal positions are controlled by position control unit. Position control unit controls positions of magnetic unit gantries and pocket gantry independently. Magnetic units are positioned above magnetic unit pockets, and are configured to move into and out of magnetic unit pockets. Magnetic unit pockets are configured to at least partially cover the lower end of magnetic units when magnetic units move into magnetic unit pockets. Well area comprises wells in a two-dimensional arrangement.

In some embodiments, each the magnetic unit has a rod-like shape. In some embodiments, each magnetic unit pocket has a closed-bottom cylindrical shape. In some embodiments, the magnetic unit pockets are configured to move or oscillate vertically relative to the one more well sets, to facilitate mixing of the buffer and microcarriers in the wells. In some embodiments, the one or more magnetic unit pockets are reusable. In some embodiments, the one or more magnetic unit pockets are disposable. In some embodiments, the one or more magnetic unit pockets comprise plastic.

In some aspects, a microcarrier of the present disclosure comprises a capture agent. In some embodiments, the capture agent for a particular microcarrier species may be a "unique capture agent," e.g., a capture agent is associated with a particular microcarrier species having a particular identifier (e.g., analog code). The capture agent can be any biomolecule or a chemical compound capable of binding one or more analytes (such as a biomolecule or a chemical compound) present in the solution. Examples of biomolecule capture agents comprise, but are not limited to, a DNA molecule, a DNA-analog-molecule, an RNA-molecule, an RNA-analog-molecule, an oligonucleotide, a polynucleotide, a protein, an enzyme, a lipid, a phospholipid, a carbohydrate moiety, a polysaccharide, an antigen, a virus, a cell, an antibody, a small molecule, a bacterial cell, a cellular organelle, and an antibody fragment. Examples of chemical compound capture agents comprise, but are not limited to, individual components of chemical libraries, small molecules, or environmental toxins (for example, pesticides or heavy metals). In some embodiments, the capture agents comprise a nucleotide. In some embodiments, the capture agents comprise an antigen. In some embodiments, the capture agents comprise an antibody.

In some embodiments, the capture agent is coupled to a surface of the microcarrier (in some embodiments, in at least a center portion of the microcarrier surface). In some embodiments, the capture agent can be chemically attached to the microcarrier. In other embodiments, the capture agent can be physically absorbed to the surface of the microcarrier. In some embodiments, the attachment linkage between the capture agent and the microcarrier surface can be a covalent bond. In other embodiments, the attachment linkage between the capture agent and the microcarrier surface can be a non-covalent bond comprising, but not limited to, a salt bridge or other ionic bond, one or more hydrogen bonds, hydrophobic interactions, Van der Waals force, London dispersion force, a mechanical bond, one or more halogen bonds, aurophilicity, intercalation, or stacking.

In some aspects, more than one (such as two, three, four, five, six, seven, eight, nine, or ten) capture agents for the same analyte can each be associated with a microcarrier described herein. In this embodiment, each capture agent for a particular analyte binds to the analyte with a different affinity as measured by the dissociation constant of analyte/capture agent binding. Accordingly, within a plurality of microcarriers in a composition, there can be two or more subpopulations of microcarriers with capture agents that bind to the same analyte, but wherein the capture agents associated with each subpopulation bind to the analyte with a different affinity. In some embodiments, the dissociation constant of the analyte for any of the capture agents is not greater than 10⁻⁶ M, such as 10⁻⁷M or 10⁻⁸M. In other embodiments, the dissociation constant of the analyte for any of the capture agents is from about 10⁻¹⁰ M to about 10⁻⁶ M, such from about 10⁻¹⁰ M to about 10⁻⁷ M, about 10⁻¹⁰ M to about 10⁻⁸ M, about 10⁻¹⁰ M to about 10⁻⁹ M, about 10⁻⁹ M to about 10⁻⁶ M, about 10⁻⁹ M to about 10⁻⁷ M, about 10⁻⁹ M to about 10⁻⁸ M, about 10⁻⁸ M to about 10⁻⁶ M, or about 10⁻⁸ M to about 10⁻⁷ M. In some embodiments, the dissociation constant of the analyte for any two capture agents differs by as much as about 3 log₁₀, such as by as much as about 2.5 log₁₀, 2 log₁₀, 1.5 log₁₀, or 1 logic.

In some embodiments, an analyte of the present disclosure is coupled to a microcarrier for the capture of one or more analytes. In some embodiments, the one or more analytes may be captured from a sample, such as a biological sample described herein. In some embodiments, an analyte may comprise without limitation a DNA molecule, a DNA-analog-molecule, an RNA-molecule, an RNA-analog-molecule, a polynucleotide, a protein, an enzyme, a lipid, a phospholipid, a carbohydrate moiety, a polysaccharide, an antigen, a virus, a cell, an antibody, a small molecule, a bacterial cell, a cellular organelle, and an antibody fragment. In other embodiments, the analyte is a chemical compound (such as a small molecule chemical compound) capable of binding to the capture agent such as individual components of chemical libraries, small molecules, or environmental toxins.

In some embodiments, the one or more capture agents and the one or more analytes are each independently selected from the group consisting of polynucleotides. In some embodiments, the one or more capture agents are each independently selected from the group consisting of antibodies and antibody fragments, and the one or more analytes are each independently selected from the group consisting of antigens. In some embodiments, the one or more capture agents are each independently selected from the group consisting of antigens, and the one or more analytes are each independently selected from the group consisting of antibodies.

In some aspects, the analytes in a sample (such as a biological sample) can be labeled with a labeling molecule capable of emitting a detectable signal upon binding to the capture agent. In some embodiments, the labeling molecule can be colorimetric based. In other embodiments, the labeling molecule can be fluorescence-based comprising, but not limited to, phycoerythrin, blue fluorescent protein, green fluorescent protein, yellow fluorescent protein, cyan fluorescent protein, and derivatives thereof. In other embodiments, the labeling molecule can be radioisotope based, comprising, but not limited to, molecules labeled with ³²P, ³³P, ²²Na, ³⁶Cl, ²H, ³H, ³⁵S, and ¹²³I. In other embodiments, the labeling molecule is light-based comprising, but not limited to, luciferase (e.g., chemiluminescence-based), horseradish peroxidase, alkaline phosphatase, and derivatives thereof. In some embodiments, the biomolecules or chemical compounds present in the sample can be labeled with the labeling molecule prior to contact with the microcarrier. In other embodiments, the biomolecules or chemical compounds present in the sample can be labeled with the labeling molecule subsequent to contact with the microcarrier. In some embodiments, the labeling molecules comprise a label binding moiety coupled to a reporting moiety. In some embodiments, the label binding moiety is streptavidin (SA). In some embodiments the label binding moiety is an anti-biotin antibody. In some embodiments, the reporting moiety is phycoerythrin (PE). In some embodiments, the reporting moiety is a fluorescein isothiocyanate (FITC).

### Modules for molecular assays

In some embodiments, the magnetic modules provided herein are configured to perform molecular assays.

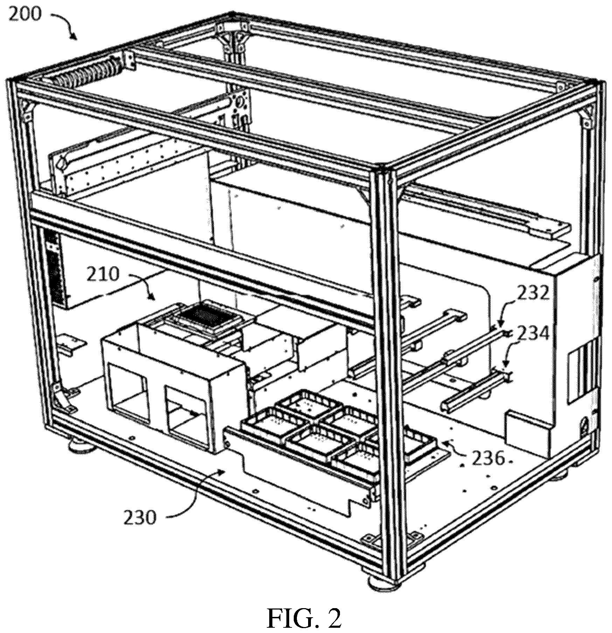

FIG. 2. depicts exemplary assay system **200,** comprising PCR module **210** and magnetic module **230.** Magnetic module **230** comprises three magnetic unit gantries **232,** three pocket gantries **234,** and well area **236.** The well area comprises six rectangular subareas, each of which can accommodate a multi-well plate pre-filled with buffers. In some embodiments, a cross-section or the front view of a gantry has an upside-down C-shape to form a C-shaped rail. In some embodiments, the upper end of the magnetic unit is T-shaped. In some embodiments, the upper end of the magnetic unit pockets is T-shaped. In some embodiments, T-shaped magnetic units or magnetic unit pockets can be inserted into the corresponding C-shaped rail. In some embodiments, the gantry comprises a C-shaped rail. In some embodiments, disposable T-shaped magnetic unit pockets can easily be inserted into or moved out of the C-shaped rail of the pocket gantry.

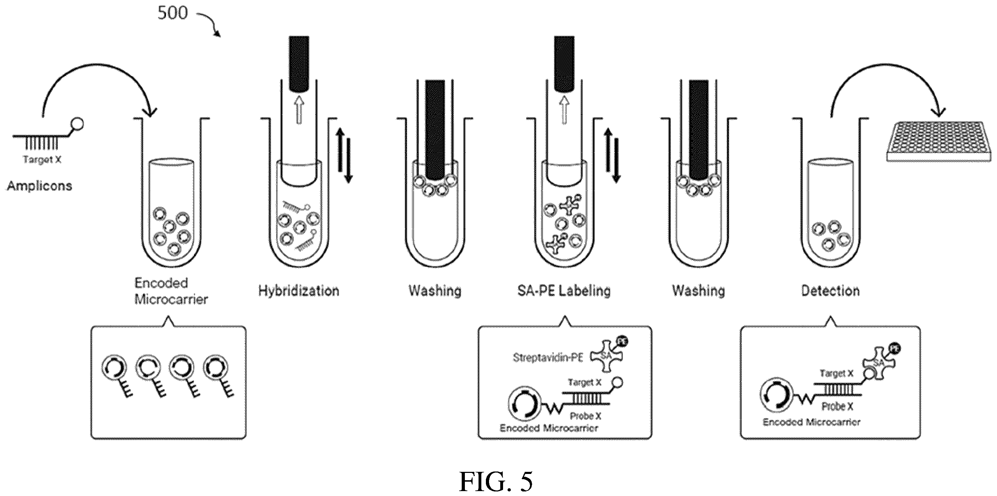

FIG. 5 depicts exemplary molecular assay process **500.** A sample containing biotin-labeled amplicons (biotin-labeled target X; amplicon can be DNA molecules amplified through PCR using biotin-labeled primers) is added to the reaction well containing microcarriers coupled to a capture agent comprising probe X (FIG. 5, "Encoded Microcarrier"). Magnetic unit pocket moves into the reaction well, and moves, moves up and down, and/or vibrates to facilitate mixing of the amplicons with the microcarriers, and hybridization of target X and probe X (FIG. 5, "Hybridization"). Magnetic unit moves into the reaction well and into the magnetic unit pocket, to attract the microcarriers to the exterior surface of the magnetic unit pocket. Magnetic unit and the magnetic unit pocket are transferred to a reaction wash well for washing (FIG. 5, first "Washing"). The magnetic unit moves out of the reaction well to release microcarriers into the wash buffer. The magnetic unit pocket moves, moves up and down, and/or vibrates to facilitate mixing in the wash well. Magnetic unit moves back into the magnetic unit pocket, to attract the microcarriers to the exterior surface of the magnetic unit pocket. Magnetic unit and the magnetic unit pocket moves to a labeling well to transfer the microcarriers to the labeling well. Magnetic unit moves out of the labeling well to release microcarriers into the labeling buffer, and magnetic unit pocket moves, moves up and down, and/or vibrates to facilitate mixing of microcarriers and labeling molecules (streptavidin-phycoerythrin; SA-PE) in the well (FIG. 5, "SA-PE Labeling"). Magnetic unit moves into the labeling well and into the magnetic unit pocket, to attract the microcarriers to the exterior surface of the magnetic unit pocket. Magnetic unit and the magnetic unit pocket, with the attracted microcarriers, are transferred to a labeling wash well for washing (FIG. 5, second "Washing"). After washing, microcarriers are transferred to a detection well for optical reading of the encoded microcarriers and detection of fluorescence signal from PE coupled to the microcarrier. In some variations, the washing step is repeated in multiple wash wells.

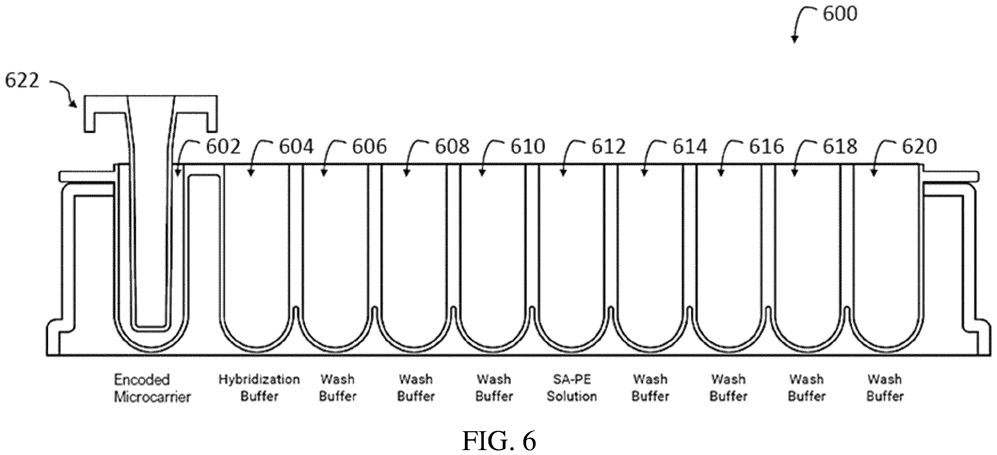

FIG. 6 depicts a cross-sectional view of exemplary well set **600,** comprising microcarrier well **602,** hybridization buffer well **604,** first reaction wash well **606,** second reaction wash well **608,** last reaction wash well **610,** labeling well **612,** first labeling wash well **614,** second labeling wash well **616,** third labeling wash well **618,** and last labeling wash well **620.** A cross-sectional view of exemplary T-shaped magnetic unit pocket **622** is also depicted. In some embodiments, encoded microcarriers are initially contained in the microcarrier well in dry form, and suspended by transferring sample (e.g., amplicon sample) to the microcarrier well and/or by transferring hybridization buffer from the hybridization buffer well to the microcarrier well using the liquid handling module. In such case, the microcarrier well can be the reaction well.

### Modules for immunoassay

In some embodiments, the magnetic module provided herein is configured to perform an immunoassay. In some embodiments, a well set in the magnetic module further comprises an antibody well. In some embodiments, the antibody well contains secondary antibodies in an antibody buffer. In some embodiments, the secondary antibodies are coupled to one or more secondary binding moieties. In some embodiments, the secondary antibodies can bind to one or more analytes. In some embodiments, each of the well set in the magnetic module further comprises one or more antibody wash wells comprising a first antibody wash well and a last antibody wash well each containing an antibody wash buffer. In some embodiments, the first antibody wash well and the last antibody wash well are the same well when there is only one antibody wash well in the set.

The wells in the well sets may be arranged in any suitable order. In some embodiments, the reaction well, one or more reaction wash wells, antibody well, one or more antibody wash wells, labeling well, and one or more labeling wash wells are positioned along a straight line in such order. In some embodiments, the reaction well, one or more reaction wash wells, antibody well, one or more antibody wash wells, labeling well, and one or more labeling wash wells are positioned along a curved line in such order. In some embodiments, the number of the one or more magnetic units and the number of antibody wells are the same.

In some embodiments, the secondary antibodies are selected from the group consisting of antibodies and antibody fragments. In some embodiments, the one or more secondary binding moieties can bind to labeling molecules. In some embodiments, the one or more secondary binding moieties comprise biotin.

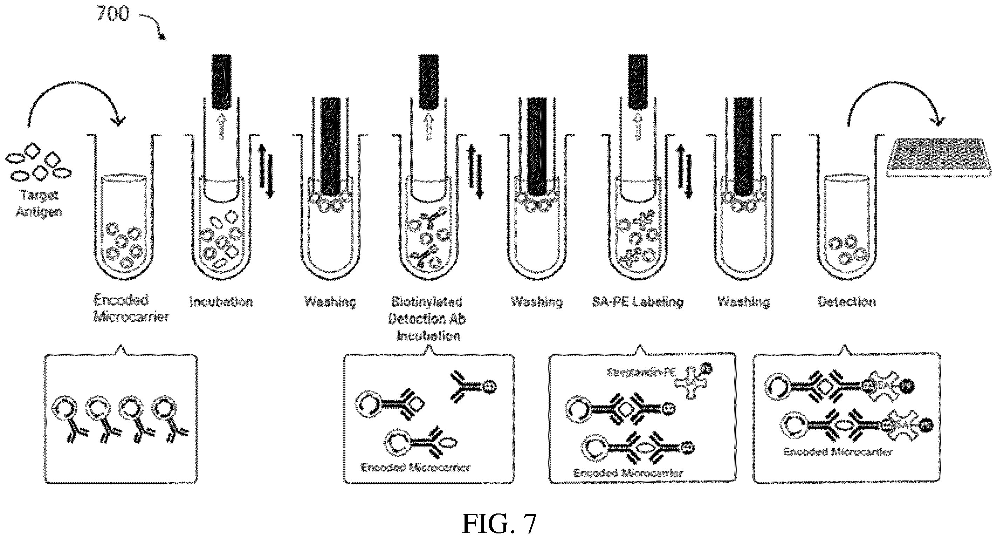

FIG. 7 depicts exemplary immunoassay process **700** for detecting antigens in a sample. A sample containing antigens is added to the reaction well containing microcarriers coupled to a capture agent comprising antibodies that can bind to target antigens (FIG. 7, "Encoded Microcarrier"). Magnetic unit pocket moves into the reaction well, and moves, moves up and down, and/or vibrates to facilitate mixing of the antigens with the microcarriers (FIG. 7, "Incubation"). Magnetic unit moves into the reaction well and into the magnetic unit pocket, to attract the microcarriers to the exterior surface of the magnetic unit pocket. Magnetic unit and the magnetic unit pocket are transferred to a reaction wash well for washing (FIG. 7, first "Washing"). The magnetic unit moves out of the reaction well to release microcarriers into the wash buffer. The magnetic unit pocket moves, moves up and down, and/or vibrates to facilitate mixing in the well. Magnetic unit moves back into the magnetic unit pocket, to attract the microcarriers to the exterior surface of the magnetic unit pocket. Magnetic unit and the magnetic unit pocket moves to an antibody well. Magnetic unit moves out of the antibody well to release microcarriers into the antibody buffer, and magnetic unit pocket moves, moves up and down, and/or vibrates to facilitate mixing of microcarriers and secondary antibodies in the well (FIG. 7, "Biotinylated Detection Ab Incubation"). Secondary antibodies bind to the antigens bound to microcarriers through their capture agents. Magnetic unit moves into the antibody well and into the magnetic unit pocket, to attract the microcarriers to the exterior surface of the magnetic unit pocket. Magnetic unit and the magnetic unit pocket are transferred to an antibody wash well for washing (FIG. 7, second "Washing"). The magnetic unit moves out of the antibody wash well to release microcarriers into the wash buffer. The magnetic unit pocket moves, moves up and down, and/or vibrates to facilitate mixing in the wash well. Magnetic unit moves back into the magnetic unit pocket, to attract the microcarriers to the exterior surface of the magnetic unit pocket. Magnetic unit and the magnetic unit pocket moves to a labeling well to transfer the microcarriers to the labeling well. Magnetic unit moves out of the labeling well to release microcarriers into the labeling buffer, and magnetic unit pocket moves, moves up and down, and/or vibrates to facilitate mixing of microcarriers and labeling molecules (streptavidin-phycoerythrin; SA-PE) in the well (FIG. 7, "SA-PE Labeling"). Magnetic unit moves into the labeling well and into the magnetic unit pocket, to attract the microcarriers to the exterior surface of the magnetic unit pocket. Magnetic unit and the magnetic unit pocket, with the attracted microcarriers, are transferred to a labeling wash well for washing (FIG. 7, third "Washing"). After washing, microcarriers are transferred to a detection well for optical reading of the encoded microcarriers and detection of fluorescence signal from the labeling molecules coupled to the microcarrier. In some variations, the washing step is repeated in multiple wash wells.

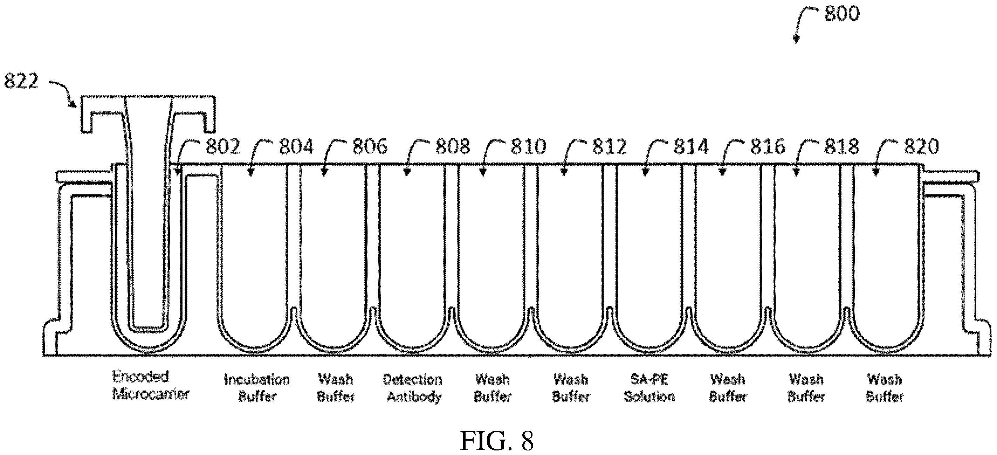

FIG. 8 depicts a cross-sectional view of exemplary well set **800,** comprising microcarrier well **802,** incubation buffer well **804,** reaction wash well **806,** antibody well **808,** first antibody wash well **810,** last antibody wash well **812,** labeling well **814,** first labeling wash well **816,** second labeling wash well **818,** and last labeling wash well **820.** A cross-sectional view of exemplary T-shaped magnetic unit pocket **822** is also depicted. In some embodiments, encoded microcarriers are initially contained in the microcarrier well in dry form, and suspended by transferring sample (e.g., antigen sample) to the microcarrier well and/or by transferring incubation buffer from the incubation buffer well to the microcarrier well using the liquid handling module. In such case, the microcarrier well can be the reaction well.

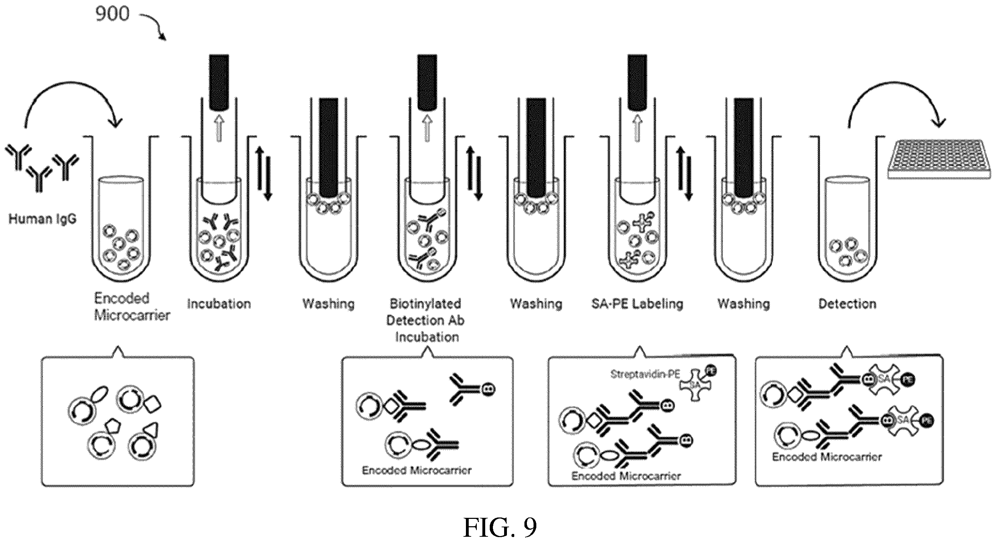

FIG. 9 depicts exemplary immunoassay process 900 for detecting antibodies in a sample. A sample containing antibodies (Human IgG in this case) is added to the reaction well containing microcarriers coupled to a capture agent comprising antigens to which antibodies in the sample can bind (FIG. 9, "Encoded Microcarrier"). Magnetic unit pocket moves into the reaction well, and moves, moves up and down, and/or vibrates to facilitate mixing of the antibodies with the microcarriers (FIG. 7, "Incubation"). Magnetic unit moves into the reaction well and into the magnetic unit pocket, to attract the microcarriers to the exterior surface of the magnetic unit pocket. Magnetic unit and the magnetic unit pocket are transferred to a reaction wash well for washing (FIG. 7, first "Washing"). The magnetic unit moves out of the reaction well to release microcarriers into the wash buffer. The magnetic unit pocket moves, moves up and down, and/or vibrates to facilitate mixing in the well. Magnetic unit moves back into the magnetic unit pocket, to attract the microcarriers to the exterior surface of the magnetic unit pocket. Magnetic unit and the magnetic unit pocket moves to an antibody well. Magnetic unit moves out of the antibody well to release microcarriers into the antibody buffer, and magnetic unit pocket moves, moves up and down, and/or vibrates to facilitate mixing of microcarriers and secondary antibodies in the well (FIG. 9, "Biotinylated Detection Ab Incubation"). Secondary antibodies bind to the sample antigens bound to microcarriers through their capture agents. Magnetic unit moves into the antibody well and into the magnetic unit pocket, to attract the microcarriers to the exterior surface of the magnetic unit pocket. Magnetic unit and the magnetic unit pocket are transferred to an antibody wash well for washing (FIG. 9, second "Washing"). The magnetic unit moves out of the antibody wash well to release microcarriers into the wash buffer. The magnetic unit pocket moves, moves up and down, and/or vibrates to facilitate mixing in the wash well. Magnetic unit moves back into the magnetic unit pocket, to attract the microcarriers to the exterior surface of the magnetic unit pocket. Magnetic unit and the magnetic unit pocket moves to a labeling well to transfer the microcarriers to the labeling well. Magnetic unit moves out of the labeling well to release microcarriers into the labeling buffer, and magnetic unit pocket moves, moves up and down, and/or vibrates to facilitate mixing of microcarriers and labeling molecules (streptavidin-phycoerythrin; SA-PE) in the well (FIG. 9, "SA-PE Labeling"). Magnetic unit moves into the labeling well and into the magnetic unit pocket, to attract the microcarriers to the exterior surface of the magnetic unit pocket. Magnetic unit and the magnetic unit pocket, with the attracted microcarriers, are transferred to a labeling wash well for washing (FIG. 9, third "Washing"). After washing, microcarriers are transferred to a detection well for optical reading of the encoded microcarriers and detection of fluorescence signal from the labeling molecules coupled to the microcarrier. In some variations, the washing step is repeated in multiple wash wells.

### Encoded Microcarriers

The magnetic modules provided herein comprise one or more encoded microcarriers. Encoded microcarriers are described in detail in PCT/IB2016/000937, PCT/US2016/063202 and PCT/US2022/031049, the full contents of which are incorporated herein by reference.

In some aspects, provided herein are magnetic modules for reacting or labeling encoded microcarriers. In some embodiments, the encoded microcarriers comprise a substantially transparent magnetic polymer layer having a first surface and a second surface, the first and the second surfaces being parallel to each other, wherein the substantially transparent magnetic polymer comprises a mixture of a substantially transparent polymer and a plurality of magnetic nanoparticles, and wherein the magnetic nanoparticles comprise iron(II,III) oxide or iron(III) oxide. In some embodiments, the encoded microcarriers further comprise a substantially non-transparent layer, wherein the substantially non-transparent layer is affixed to the first surface of the substantially transparent magnetic polymer layer, and wherein an outline of the substantially non-transparent layer constitutes a two-dimensional shape representing an analog code. In some embodiments, the encoded microcarriers further comprise one or more capture agents for capturing one or more analytes, wherein the one or more capture agents are coupled to at least one of the first surface and the second surface of the substantially transparent magnetic polymer layer. In some embodiments, the magnetic component of the encoded microcarriers comprise the substantially transparent magnetic polymer layer. Configurations, parameters, and optional features of the encoded microcarriers of the present disclosure are provided in the non-limiting descriptions infra.

In some embodiments, a substantially transparent magnetic polymer layer of the present disclosure comprises a mixture of a substantially transparent polymer and a plurality of magnetic nanoparticles. In some embodiments, the substantially transparent magnetic polymer layer comprises a suspension of magnetic nanoparticles in a substantially transparent polymer, e.g., an epoxy-based polymer. For example, the magnetic nanoparticles can be made into a stable dispersion (e.g., with the use of a solvent comprising but not limited to cyclopentanone or g-Butyrolactone, and/or a dispersing agent comprising but not limited to phosphoric acid polymers) and mixed with a dissolved polymer or monomeric form of the polymer. Incorporating magnetic properties into the substantially transparent polymer layer has advantages over using a dedicated magnetic layer (such as, e.g., a magnetic ring) by allowing for a reduced overall size (e.g., area) of the microcarriers, thereby increasing the number of microcarriers that can be produced from a single wafer and reducing the per-unit cost. In addition, a magnetic substantially transparent polymer layer does not need to be sandwiched between polymer layers to insulate it from the environment, as other dedicated magnetic layers (e.g., nickel layers) may require. This simplified, two-layer model reduces manufacturing costs and increases manufacturing consistency without sacrificing functionality. Exemplary descriptions and techniques related to magnetic nanoparticle suspensions and mixing the same with epoxy-based polymers may be found, *e.g.,* in Suter, Marcel. Photopatternable superparamagnetic nanocomposite for the fabrication of microstructures. Diss. ETH Zurich, 2011; and Suter, M. (2011) Sensors and Actuators B: Chemical 156:433-43.

In some embodiments, the magnetic nanoparticles are superparamagnetic nanoparticles. Without wishing to be bound to theory, it is thought that such a material is advantageous for microcarrier fabrication because the resulting microcarriers display no residual magnetism and/or low magnetic attraction with each other (as opposed to magnetic materials with some residual magnetism after removal of the magnetic field, such as nickel), thus preventing them from interacting detrimentally with each other in solution. It is also thought that such materials are uniquely suited for analog encoding (*e.g.,* as described herein) because the materials (*e.g.,* an SU-8/ iron(II,III) oxide or iron(III) oxide nanoparticle mixture or suspension, which can replace the use of nickel or rare earth metals) allow for very thin magnetic layers with enhanced image recognizability (*e.g.,* when imaged as described herein). These microcarriers are also thought to be simpler for fabrication. In certain embodiments, the magnetic nanoparticles comprise iron (III) oxide (Fe₂O₃; also known as ferric oxide or maghemite) and/or iron(II,III) oxide (Fe₃O₄; also known as magnetite). In certain embodiments, the epoxy-based polymer is SU-8.

In some embodiments, magnetic nanoparticles of the present disclosure are less than about 30 nm in diameter. In some embodiments, magnetic nanoparticles of the present disclosure are greater than or equal to about 3 nm in diameter. In some embodiments, magnetic nanoparticles of the present disclosure can be any size (e.g., diameter) less than about any of the following sizes (in nm): 30, 25, 20, 15, 10, or 5. In some embodiments, magnetic nanoparticles of the present disclosure can be any size (e.g., diameter) greater than or equal to about any of the following sizes (in nm): 3, 5, 10, 15, 20, or 25. That is, the magnetic nanoparticles can be of any size (e.g., diameter) having an upper limit of 30, 25, 20, 15, 10, or 5 nm and an independently selected lower limit of 3, 5, 10, 15, 20, or 25 nm, wherein the lower limit is less than the upper limit.

In some embodiments, a substantially transparent magnetic polymer layer comprises a mixture of a substantially transparent polymer and a plurality of magnetic nanoparticles, wherein the plurality of magnetic nanoparticles comprises less than about 10% (by weight) and/or greater than about 0.1% (by weight) of the mixture. In some embodiments, the plurality of magnetic nanoparticles comprises around 2.5% (by weight) of the mixture. In some embodiments, a substantially transparent magnetic polymer layer of the present disclosure comprises a mixture comprising a plurality of magnetic nanoparticles at a concentration less than about any of the following concentrations (% by weight): 10, 9, 8, 7, 6, 5, 4, 3, 2, 1, 0.9, 0.8, 0.7, 0.6, 0.5, 0.4, or 0.3. In some embodiments, a substantially transparent magnetic polymer layer of the present disclosure comprises a mixture comprising a plurality of magnetic nanoparticles at a concentration greater than about any of the following concentrations (% by weight): 0.1, 0.2, 0.3, 0.4, 0.5, 0.6, 0.7, 0.8, 0.9, 1, 2, 3, 4, 5, 6, 7, or 8. That is, a substantially transparent magnetic polymer layer of the present disclosure can comprise a mixture comprising a plurality of magnetic nanoparticles at a concentration having an upper limit of 10, 9, 8, 7, 6, 5, 4, 3, 2, 1, 0.9, 0.8, 0.7, 0.6, 0.5, 0.4, or 0.3% and an independently selected lower limit of 0.1, 0.2, 0.3, 0.4, 0.5, 0.6, 0.7, 0.8, 0.9, 1, 2, 3, 4, 5, 6, 7, or 8%, wherein the lower limit is less than the upper limit.

In some embodiments, a substantially transparent polymer of the present disclosure comprises an epoxy-based polymer. Suitable epoxy-based polymers for fabrication of the compositions described herein comprise, but are not limited to, the EPON^{™} family of epoxy resins provided by Hexion Specialty Chemicals, Inc. (Columbus, OH) and any number of epoxy resins provided by The Dow Chemical Company (Midland, MI). Many examples of suitable polymers are commonly known in the art, comprising without limitation SU-8, EPON 1002F, EPON 165/154, and a poly(methyl methacrylate)/poly(acrylic acid) block copolymer (PMMA-co-PAA). For additional polymers, see, for example, Warad, IC Packaging: Package Construction Analysis in Ultra Small IC Packaging, LAP LAMBERT Academic Publishing (2010); The Electronic Packaging Handbook, CRC Press (Blackwell, ed.), (2000); and Pecht et al., Electronic Packaging Materials and Their Properties, CCR Press, 1st ed., (1998). These types of materials have the advantage of not swelling in aqueous environments which ensures that uniform microcarrier size and shape are maintained within the population of microcarriers. In some embodiments, the substantially transparent polymer is a photoresist polymer. In some embodiments, the epoxy-based polymer is an epoxy-based, negative-tone, near-UV photoresist. In some embodiments, the epoxy-based polymer is SU-8.

In some embodiments, a microcarrier of the present disclosure comprises a substantially transparent magnetic polymer layer that is greater than or equal to about 0.1 µm in thickness. In some embodiments, a microcarrier of the present disclosure comprises a substantially transparent magnetic polymer layer that is less than or equal to about 50 µm in thickness. In some embodiments, a microcarrier of the present disclosure comprises a substantially transparent magnetic polymer layer that is between about 0.1 µm and about 50 µm in thickness. In some embodiments, a microcarrier of the present disclosure comprises a substantially transparent magnetic polymer layer with a thickness that is less than or equal to about any of the following thicknesses (in µm): 50, 45, 40, 35, 30, 25, 20, 15, 10, 9, 8, 7, 6, 5, 4, 3, 2, 1, 0.9, 0.8, 0.7, 0.6, 0.5, 0.4, or 0.3. In some embodiments, a microcarrier of the present disclosure comprises a substantially transparent magnetic polymer layer with a thickness that is greater than or equal to about any of the following thicknesses (in µm): 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, or 45. That is, a microcarrier of the present disclosure can comprise a substantially transparent magnetic polymer layer with a thickness having an upper limit of 50, 45, 40, 35, 30, 25, 20, 15, 10, 9, 8, 7, 6, 5, 4, 3, 2, 1, 0.9, 0.8, 0.7, 0.6, 0.5, 0.4, or 0.3 µm and an independently selected lower limit of 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, or 45 µm, wherein the lower limit is less than the upper limit.

In some embodiments, a microcarrier of the present disclosure comprises a substantially non-transparent layer. For example, the substantially non-transparent layer can be made of a substantially non-transparent polymer, or a metal.

In some embodiments, the substantially non-transparent layer comprises a polymer described herein (e.g., SU-8) mixed with one or more non-transparent or colored dye(s). In other embodiments, the substantially non-transparent layer comprises a black matrix resist. Any black matrix resist known in the art may be used; see, e.g., U.S. Patent No. 8,610,848 for exemplary black matrix resists and methods related thereto. In some embodiments, the black matrix resist may be a photoresist colored with a black pigment, e.g., as patterned on the color filter of an LCD as part of a black matrix. Black matrix resists may comprise without limitation those sold by Toppan Printing Co. (Tokyo), Tokyo OHKA Kogyo (Kawasaki), and Daxin Materials Corp. (Taichung City, Taiwan).

In some embodiments, the substantially non-transparent layer comprises a substantially non-transparent polymer that exhibits an absorbance of greater than about 1.8 (OD) at a wavelength between about 230 nm and about 660 nm. For example, the polymer can exhibit an absorbance of greater than about 1.8 (OD) at one or more wavelengths selected from the group consisting of 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, and 660 nm.

In some embodiments, the substantially non-transparent layer comprises a metal. In some embodiments, the metal lacks residual magnetism. In some embodiments, the substantially non-transparent layer comprises nickel, titanium, copper, and/or chromium.

In some embodiments, a microcarrier of the present disclosure comprises a substantially non-transparent layer that is greater than or equal to about 0.05 µm in thickness. In some embodiments, a microcarrier of the present disclosure comprises a substantially non-transparent layer that is less than or equal to about 2 µm in thickness. In some embodiments, a microcarrier of the present disclosure comprises a substantially non-transparent layer that is between about 0.05 µm and about 2 µm in thickness. In some embodiments, a microcarrier of the present disclosure comprises a substantially non-transparent layer with a thickness that is less than or equal to about any of the following thicknesses (in µm): 2, 1.9, 1.8, 1.7, 1.6, 1.5, 1.4, 1.3, 1.2, 1.1, 1.0, 0.9, 0.8, 0.7, 0.6, 0.5, 0.45, 0.4, 0.35, 0.3, 0.25, 0.2, 0.15, 0.125, 0.1, or 0.075. In some embodiments, a microcarrier of the present disclosure comprises a substantially non-transparent layer with a thickness that is greater than or equal to about any of the following thicknesses (in µm): 0.05, 0.075, 0.1, 0.125, 0.15, 0.2, 0.25, 0.3, 0.35, 0.4, 0.45, 0.5, 0.6, 0.7, 0.8, 0.9, 1.0, 1.1, 1.2, 1.3, 1.4, 1.5, 1.6, 1.7, or 1.8. That is, a microcarrier of the present disclosure can comprise a substantially non-transparent layer with a thickness having an upper limit of 2, 1.9, 1.8, 1.7, 1.6, 1.5, 1.4, 1.3, 1.2, 1.1, 1.0, 0.9, 0.8, 0.7, 0.6, 0.5, 0.45, 0.4, 0.35, 0.3, 0.25, 0.2, 0.15, 0.125, 0.1, or 0.075 µm and an independently selected lower limit of 0.05, 0.075, 0.1, 0.125, 0.15, 0.2, 0.25, 0.3, 0.35, 0.4, 0.45, 0.5, 0.6, 0.7, 0.8, 0.9, 1.0, 1.1, 1.2, 1.3, 1.4, 1.5, 1.6, 1.7, or 1.8 µm, wherein the lower limit is less than the upper limit.

In some embodiments, the analog code comprises one or more overlapping or partially overlapping arc elements forming a continuous or discontinuous ring (e.g., surrounding a center portion of the microcarrier). The two-dimensional shape is decoded by imaging the microcarrier (e.g., by light microscopy), such that an image of the code is formed by the pattern generated by light passed through the substantially transparent magnetic polymer layer and light blocked from passing through the substantially non-transparent layer. A non-limiting example of a two-dimensional shape made of overlapping arc elements that form a discontinuous ring is illustrated in PCT/US2022/031049.

In some embodiments, a microcarrier of the present disclosure may be encoded with a substantially non-transparent layer that constitutes a two-dimensional shape. For example, as described above, the two-dimensional shape may constitute the shape of a substantially non-transparent layer that contrasts with a substantially transparent layer of the microcarrier, or it may constitute the shape of the microcarrier itself (e.g., the perimeter). That is to say, the code is the shape of the substantially non-transparent layer itself (e.g., rather than a code generated by a fluorescent or other visible moiety on the surface of a layer of the microcarrier). Any two-dimensional shape that can encompass a plurality of resolvable and distinctive varieties may be used. In some embodiments, the two-dimensional shape comprises one or more of linear, circular, elliptical, rectangular, quadrilateral, or higher polygonal aspects, elements, and/or shapes.

In some embodiments, the microcarrier further comprises an orientation indicator for orienting the analog code of the substantially non-transparent layer. Any feature of the microcarrier that is visible and/or detectable by imaging (e.g., a form of microscopic or other imaging described herein) and/or by image recognition software may serve as an orientation indicator. An orientation indicator may serve as a point of reference, e.g., for an image recognition algorithm, to orient the image of an analog code in a uniform orientation (i.e., the shape of the substantially non-transparent layer). Advantageously, this simplifies image recognition, as the algorithm would only need to compare the image of a particular analog code against a library of analog codes in the same orientation, and not against a library comprising all analog codes in all possible orientations. In some embodiments, the orientation indicator comprises an asymmetry of the substantially non-transparent layer, such as a discontinuity of its outline or shape. For example, the orientation indicator may comprise a visible feature, such as an asymmetry, of the two-dimensional shape representing the analog code of the microcarrier.

In some embodiments, a microcarrier of the present disclosure is a substantially circular disc. As used herein, a substantially circular shape may refer to any shape having a roughly identical distance between all of the points of the shape's perimeter and the shape's geometric center. In some embodiments, a shape is considered to be substantially circular if the variation among any of the potential radii connecting the geometric center and a given point on the perimeter exhibit 10% or lesser variation in length. As used herein, a substantially circular disc may refer to any substantially circular shape wherein the thickness of the shape is significantly less than its diameter. For example, in some embodiments, the thickness of a substantially circular disc may be less than about 50%, less than about 40%, less than about 30%, less than about 20%, less than about 15%, less than about 10%, or less than about 5% of its diameter. In certain embodiments, the thickness of the substantially circular disc may about 20% of its diameter.

In some embodiments, the microcarrier is less than about 200 µm in diameter. For example, in some embodiments, the diameter of the microcarrier is less than about 200 µm, less than about 180 µm, less than about 160 µm, less than about 140 µm, less than about 120 µm, less than about 100 µm, less than about 80 µm, less than about 60 µm, less than about 40 µm, or less than about 20 µm. In some embodiments, the diameter of the microcarrier is greater than about 5 µm, greater than about 10 µm, greater than about 20 µm, greater than about 30 µm, greater than about 40 µm, greater than about 50 µm, greater than about 60 µm, greater than about 70 µm, greater than about 80 µm, greater than about 90 µm, greater than about 100 µm, greater than about 120 µm, greater than about 140 µm, or greater than about 150 µm. In some embodiments, a microcarrier of the present disclosure can be any size (e.g., diameter) less than about any of the following sizes (in µm): 200, 180, 160, 140, 120, 100, 80, 60, 40, or 20. In some embodiments, magnetic nanoparticles of the present disclosure can be any size (e.g., diameter) greater than or equal to about any of the following sizes (in µm): 5, 10, 20, 30, 40, 50, 60, 70, 80, 90, 100, 120, 140, or 150. That is, the magnetic nanoparticles can be of any size (e.g., diameter) having an upper limit of 200, 180, 160, 140, 120, 100, 80, 60, 40, or 20 µm and an independently selected lower limit of 5, 10, 20, 30, 40, 50, 60, 70, 80, 90, 100, 120, 140, or 150 µm, wherein the lower limit is less than the upper limit.

In some embodiments, the diameter of the microcarrier is about 180 µm, about 160 µm, about 140 µm, about 120 µm, about 100 µm, about 90 µm, about 80 µm, about 70 µm, about 60 µm, about 50 µm, about 40 µm, about 30 µm, about 20 µm, or about 10 µm. In certain embodiments, the microcarrier is about 40 µm in diameter.

In some embodiments, the microcarrier is less than about 50 µm in thickness. For example, in some embodiments, the thickness of the microcarrier is less than about 70 µm, about 60 µm, about 50 µm, about 40 µm, about 30 µm, less than about 25 µm, less than about 20 µm, less than about 15 µm, less than about 10 µm, or less than about 5 µm. In some embodiments, the thickness of the microcarrier is less than about any of the following thicknesses (in µm): 50, 45, 40, 35, 30, 25, 20, 15, 10, 9, 8, 7, 6, 5, 4, 3, or 2. In some embodiments, the thickness of the microcarrier is greater than about any of the following thicknesses (in µm): 1, 2, 3, 4, 5, 6, 7, 8, 9, 10, 15, 20, 25, 30, 35, 40, or 45. That is, the thickness of the microcarrier may be any of a range of thicknesses (in µm) having an upper limit of 50, 45, 40, 35, 30, 25, 20, 15, 10, 9, 8, 7, 6, 5, 4, 3, or 2 and an independently selected lower limit of 1, 2, 3, 4, 5, 6, 7, 8, 9, 10, 15, 20, 25, 30, 35, 40, or 45, wherein the lower limit is less than the upper limit.

In some embodiments, the thickness of the microcarrier is about 50 µm, about 45 µm, about 40 µm, about 35 µm, about 30 µm, about 25 µm, about 20 µm, about 19 µm, about 18 µm, about 17 µm, about 16 µm, about 15 µm, about 14 µm, about 13 µm, about 12 µm, about 11 µm, about 10 µm, about 9 µm, about 8 µm, about 7 µm, about 6 µm, about 5 µm, about 4 µm, about 3 µm, about 2 µm, or about 1 µm. In some embodiments, the thickness of the microcarrier is between about 50 µm and about 2 µm, between about 20 µm and about 2 µm, or between about 10 µm and about 2 µm. In certain embodiments, the microcarrier is about 5 µm in thickness.

### III. Assay system

Certain aspects of the present disclosure relate to an assay system comprising any of the magnetic modules provided herein. In some embodiments, the assay system further comprises a detection module. In some embodiments, the detection module is configured to detect signal from the magnetic microcarriers. Any types of signals that is detectable can be used, such as fluorescence intensity, anisotropy, light emission, color, visible pattern, concentration, reaction product, nucleotide sequence, and polypeptide sequence. In some embodiments, the signal is a fluorescence signal.

In some embodiments, the assay system comprises one or more detection wells. In some embodiments, the one or more detection wells are positioned in the same one- or two-dimensional arrangement as each type of wells in the magnetic module. In some embodiments, the one or more detection wells are positioned in the same one- or two-dimensional arrangement as the last labeling wash wells, such that the microcarriers in the last labeling wash wells can be transferred to the one or more detection wells simultaneously.

The one or more detection wells may be in any suitable shape and material. In some embodiments, the one or more detection wells have a flat bottom. In some embodiments, the one or more detection wells are substantially transparent. In some embodiments, the one or more detection wells are substantially non-transparent.

In some embodiments, the one or more detection wells and the one or more magnetic units have the same one- or two-dimensional arrangement, such that the one or more magnetic units can simultaneously move into and out of the one or more detection wells. In some embodiments, the one or more detection wells and the one or more magnetic unit pockets have the same one- or two-dimensional arrangement, such that the one or more magnetic unit pockets can simultaneously move into and out of the one or more detection wells.

In some embodiments, the assay system further comprises a PCR module. Any PCR modules configured to perform a polymerase chain reaction (PCR) can be used. In some embodiments, the PCR module is configured to perform PCR using primers coupled to one or more secondary binding moieties that can bind to the labeling molecules. In some embodiments, the secondary binding moieties comprise a biotin.

In some embodiments, the assay system further comprises a liquid handling module. The liquid handling module is configured to transfer liquid to and from any location within or out of the assay system. For example, the liquid handling module can transfer liquid (e.g., sample or buffer) to, from and between the wells of the magnetic modules. In some embodiments, the liquid handling module is configured to transfer sample (e.g., amplicon) from the PCR module to the magnetic module (e.g., reaction well or microcarrier well).

In some embodiments, the assay system further comprises a hybridization buffer well. In some embodiments, the hybridization buffer well is within each well set. In some embodiments, the hybridization buffer well contains a hybridization buffer. In some embodiments, the liquid handling module is configured to transfer at least part of the hybridization buffer from the hybridization buffer well to the reaction well or to the microcarrier well.

In some embodiments, the assay system further comprises an incubation buffer well. In some embodiments, the incubation buffer well is within each well set. In some embodiments, the incubation buffer well contains an incubation buffer. In some embodiments, the liquid handling module is configured to transfer at least part of the incubation buffer from the incubation buffer well to the reaction well or the microcarrier well.

### IV. Methods for performing assays using the magnetic modules and assay systems

Certain aspects of the present disclosure relate to methods for carrying out multiplex assays, such as reacting or labeling magnetic microcarriers, detecting target molecules or performing immunoassays using a magnetic module described herein. Advantageously, the magnetic modules allow for assays with improved throughput with a large number of potentially unique microcarriers and reduced recognition error.

In some embodiments, the methods comprise optically reading the magnetic microcarriers. In some embodiments, optically reading the magnetic microcarriers decodes the analog code using analog shape recognition to identify the microcarrier. Conceptually, this decoding may involve imaging the analog code of each microcarrier (e.g., in a solution or sample), comparing each image against a library of analog codes, and matching each image to an image from the library, thus positively identifying the code. Optionally, as described herein, when using microcarriers that comprise an orientation indicator (e.g., an asymmetry), the decoding may further comprise a step of rotating each image to align with a particular orientation (based in part, e.g., on the orientation indicator). For example, if the orientation indicator comprises a gap, the image could be rotated until the gap reaches a predetermined position or orientation (e.g., a 0° position of the image).

Various shape recognition software, tools, and methods are known in the art. Examples of such APIs and tools comprise without limitation Microsoft^{®} Research FaceSDK, OpenBR, Face and Scene Recognition from ReKognition, Betaface API, and various ImageJ plugins. In some embodiments, the analog shape recognition may comprise without limitation image processing steps such as foreground extraction, shape detection, thresholding (e.g., automated or manual image thresholding), and the like.

It will be appreciated by one of skill in the art that the methods and microcarriers described herein may be adapted for various imaging devices, comprising without limitation a microscope, plate reader, and the like. In some embodiments, decoding the analog codes may comprise illuminating the microcarriers by passing light through the substantially transparent portions (e.g., substantially transparent polymer layer(s)) of the microcarriers and/or the surrounding solution. The light may then fail to pass through, or pass through with a lower intensity or other appreciable difference, the substantially non-transparent portions (e.g., substantially non-transparent polymer layer(s)) of the microcarriers to generate analog-coded light pattern corresponding to the microcarrier.

As described supra, any type of light microscopy may be used for the methods of the present disclosure, comprising without limitation one or more of: bright field, dark field, phase contrast, differential interference contrast (DIC), Nomarski interference contrast (NIC), Nomarski, Hoffman modulation contrast (HMC), or fluorescence microscopy. In certain embodiments, the analog codes may be decoded using bright field microscopy, and analyte(s) may be detected using fluorescence microscopy.

### Methods for molecular assay

In some embodiments, provided herein are methods of using the magnetic module or the assay system provided herein to perform or facilitate a molecular assay. In some embodiments, the method comprises: (a) transferring one or more analytes into the reaction well, wherein the reaction well contains the magnetic microcarriers in a reaction buffer. In some embodiments, the method comprises (b) moving one of the one or more magnetic unit pockets into the reaction well and moving or oscillating the magnetic unit pocket in the reaction well to mix the one or more analytes and the magnetic microcarriers. In some embodiments, the method comprises (c) moving the magnetic unit into the reaction well and into the magnetic unit pocket to attract the magnetic microcarriers to the exterior surface of the magnetic unit pocket. In some embodiments, the method comprises (d) moving the magnetic unit pocket and magnetic unit out of the reaction well and into the first reaction wash well to transfer the magnetic microcarriers from the reaction well to the first reaction wash well. In some embodiments, the method comprises (e) moving the magnetic unit pocket and magnetic unit out of the last reaction wash well and into the labeling well to transfer the magnetic microcarriers from the last reaction wash well to the labeling well. In some embodiments, the method comprises (f) moving the magnetic unit out of the labeling well while keeping the magnetic unit pocket in the labeling well, thereby releasing the magnetic microcarriers from the exterior surface of the magnetic unit pocket into the labeling buffer. In some embodiments, the method comprises (g) moving the magnetic unit into the labeling well and into the magnetic unit pocket to attract the magnetic microcarriers to the exterior surface of the magnetic unit pocket. In some embodiments, the method comprises (h) moving the magnetic unit pocket and magnetic unit out of the labeling well and into the first labeling wash well to transfer the magnetic microcarriers from the labeling well to the first labeling wash well.

It is to be appreciated that steps (a) through (h) described in the above paragraph may occur in any order, and any steps may be optionally repeated or omitted as needed. In some embodiments, steps (a) through (h) occur in the order listed above. In some embodiments, a mixing step similar to step (b) occurs after transferring and releasing microcarriers into each well, to facilitate mixing of microcarriers with buffer or reagent in each well. In some embodiments, any of the wash steps may be optionally repeated.

In some embodiments, the method further comprises moving the magnetic unit out of the first reaction wash well while keeping the magnetic unit pocket in the first reaction wash well, thereby releasing the magnetic microcarriers from the exterior surface of the magnetic unit pocket into the first reaction wash well after step (d) and before step (e). In some embodiments, the method further comprises moving or oscillating the magnetic unit pocket in the first reaction wash well to mix the magnetic microcarriers with the reaction wash buffer after step (d) and before step (e).

In some embodiments, the method further comprises moving or oscillating the magnetic unit pocket in the labeling well to mix the magnetic microcarriers with the labeling buffer after step (f) and before step (g). In some embodiments, the method further comprises, after step (h), moving the magnetic unit out of the first labeling wash well while keeping the magnetic unit pocket in the first labeling wash well, thereby releasing the magnetic microcarriers from the exterior surface of the magnetic unit pocket into the first labeling wash well. In some embodiments, the method further comprises, after step (h), moving or oscillating the magnetic unit pocket in the first labeling wash well to mix the magnetic microcarriers with the labeling wash buffer.

In some embodiments, the method further comprises, after step (h), moving the magnetic unit pocket and magnetic unit out of the last labeling wash well and into a detection well to transfer the magnetic microcarriers from the last reaction wash well to the detection well.

In some embodiments, the method further comprises moving the magnetic unit out of the detection well while keeping the magnetic unit pocket in the detection well, thereby releasing the magnetic microcarriers from the exterior surface of the magnetic unit pocket into the detection well.

In some embodiments, the method further comprises, after step (h), optically reading the magnetic microcarriers and detecting signal from labeling molecules coupled to the magnetic microcarriers.

### Methods for immunoassay

In some embodiments, provided herein are methods of using the magnetic module or the assay system provided herein to perform a molecular assay. In some embodiments, the method comprises (a) transferring one or more analytes into the reaction well, wherein the reaction well contains the magnetic microcarriers in a reaction buffer. In some embodiments, the method comprises (b) moving one of the one or more magnetic unit pockets into the reaction well and moving or oscillating the magnetic unit pocket in the reaction well to mix the one or more analytes and the magnetic microcarriers. In some embodiments, the method comprises (c) moving the magnetic unit into the reaction well and into the magnetic unit pocket to attract the magnetic microcarriers to the exterior surface of the magnetic unit pocket. In some embodiments, the method comprises (d) moving the magnetic unit pocket and magnetic unit out of the reaction well and into the first reaction wash well to transfer the magnetic microcarriers from the reaction well to the first reaction wash well. In some embodiments, the method comprises (e) moving the magnetic unit pocket and magnetic unit out of the last reaction wash well and into the antibody well to transfer the magnetic microcarriers from the last reaction wash well to the antibody well. In some embodiments, the method comprises (f) moving the magnetic unit out of the antibody well while keeping the magnetic unit pocket in the labeling well, thereby releasing the magnetic microcarriers from the exterior surface of the magnetic unit pocket into the antibody buffer. In some embodiments, the method comprises (g) moving the magnetic unit into the antibody well and into the magnetic unit pocket to attract the magnetic microcarriers to the exterior surface of the magnetic unit pocket. In some embodiments, the method comprises (h) moving the magnetic unit pocket and magnetic unit out of the antibody well and into the first antibody wash well to transfer the magnetic microcarriers from the antibody well to the first antibody wash well. In some embodiments, the method comprises (i) moving the magnetic unit pocket and magnetic unit out of the last antibody wash well and into the labeling well to transfer the magnetic microcarriers from the last antibody wash well to the labeling well. In some embodiments, the method comprises (j) moving the magnetic unit out of the labeling well while keeping the magnetic unit pocket in the labeling well, thereby releasing the magnetic microcarriers from the exterior surface of the magnetic unit pocket into the labeling buffer. In some embodiments, the method comprises (k) moving the magnetic unit into the labeling well and into the magnetic unit pocket to attract the magnetic microcarriers to the exterior surface of the magnetic unit pocket. In some embodiments, the method comprises (l) moving the magnetic unit pocket and magnetic unit out of the labeling well and into the first labeling wash well to transfer the magnetic microcarriers from the labeling well to the first labeling wash well.

It is to be appreciated that steps (a) through (l) described in the above paragraph may occur in any order, and any steps may be optionally repeated or omitted as needed. In some embodiments, steps (a) through (1) occur in the order listed. In some embodiments, a mixing step similar to step (b) occurs after transferring and releasing microcarriers into each well, to facilitate mixing of microcarriers with buffer or reagent in each well. In some embodiments, any of the wash steps may be optionally repeated.

In some embodiments, the method further comprises, after step (d) and before step (e), moving the magnetic unit out of the first reaction wash well while keeping the magnetic unit pocket in the first reaction wash well, thereby releasing the magnetic microcarriers from the exterior surface of the magnetic unit pocket into the first reaction wash well. In some embodiments, the method further comprises, after step (d) and before step (e), moving or oscillating the magnetic unit pocket in the first reaction wash well to mix the magnetic microcarriers with the reaction wash buffer.

In some embodiments, the method further comprises, after step (f) and before step (g), moving or oscillating the magnetic unit pocket in the antibody well to mix the magnetic microcarriers with the labeling buffer.

In some embodiments, the method further comprises after step (h) and before step (i), moving the magnetic unit out of the first antibody wash well while keeping the magnetic unit pocket in the first antibody wash well, thereby releasing the magnetic microcarriers from the exterior surface of the magnetic unit pocket into the first antibody wash well. In some embodiments, the method further comprises after step (h) and before step (i), moving or oscillating the magnetic unit pocket in the first antibody wash well to mix the magnetic microcarriers with the antibody wash buffer.

In some embodiments, the method further comprises after step (j) and before step (k), moving or oscillating the magnetic unit pocket in the labeling well to mix the magnetic microcarriers with the labeling buffer.

In some embodiments, the method further comprises after step (l), moving the magnetic unit out of the first labeling wash well while keeping the magnetic unit pocket in the first labeling wash well, thereby releasing the magnetic microcarriers from the exterior surface of the magnetic unit pocket into the first labeling wash well. In some embodiments, the method further comprises after step (l), moving or oscillating the magnetic unit pocket in the first labeling wash well to mix the magnetic microcarriers with the labeling wash buffer.

In some embodiments, the method further comprises after step (l), moving the magnetic unit pocket and magnetic unit out of the last labeling wash well and into a detection well to transfer the magnetic microcarriers from the last reaction wash well to the detection well.

In some embodiments, the method further comprises moving the magnetic unit out of the detection well while keeping the magnetic unit pocket in the detection well, thereby releasing the magnetic microcarriers from the exterior surface of the magnetic unit pocket into the detection well.

In some embodiments, the method further comprises after step (l), optically reading the magnetic microcarriers and detecting signal from labeling molecules coupled to the magnetic microcarriers

### EXAMPLES

The presently disclosed subject matter will be better understood by reference to the following Examples, which are provided as exemplary of the invention, and not by way of limitation.

### Example 1: Exemplary molecular assay using the magnetic modules

An exemplary molecular assay was carried out using the magnetic module described herein. A control molecular assay experiment using a conventional method was performed, where encoded microcarriers were processed in a single well using dispensing and suction channels to add or remove buffers in the well, and using shaker table to mix the microcarriers with different buffers. Median fluorescence intensity (MFI) was measured for the molecular assay using the magnetic module described herein and the control experiment. The background signal was used to monitor removal of excess SA-PE through the wash process. The results are summarized in Table 1. The small difference in the average signal MFI of less than 1.3%, calculated as (107724-106377)/106377, and the small difference in the background signal of less than 0.13%, calculated as (1596-1594)/1596, indicate that the performance of the magnetic module is comparable to that of the conventional single-well method, while achieving greater simplicity in the process and device design.

**Table 1. Molecular assay results using the magnetic modules and single-well method**

| Experiment No. | Magnetic module | Single-well method |
|---|---|---|
| 1 | 107649 | 108248 |
| 2 | 105105 | 107200 |
| Average | 106377 | 107724 |
| Background | 1596 | 1594 |

### Example 2: Exemplary immunoassay using the magnetic modules

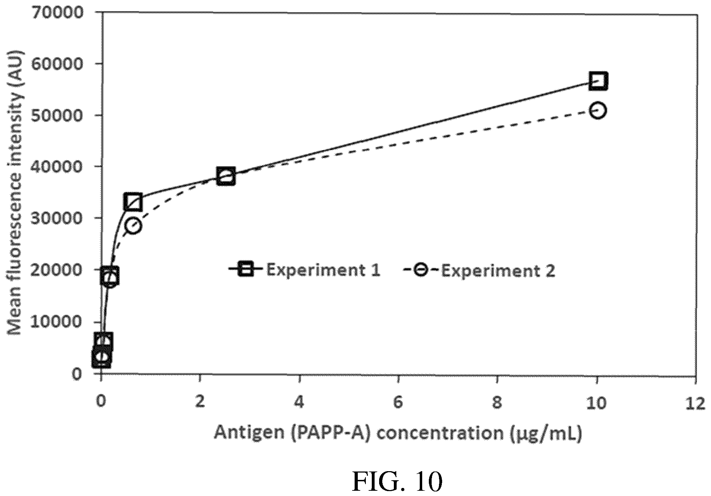

An exemplary immunoassay was performed using the magnetic module described herein. Median fluorescence intensity (MFI) was measured for the immunoassay at various antigen (pregnancy-associated plasma protein A, PAPP-A) concentrations. Results are summarized in Table 2 below and in FIG. 10. The results show that the assay system using the magnetic module is capable of generating higher signals as the antigen concentration increases. The similar MFI between experiment 1 and experiment 2 at the same antigen concentration indicates that the assay system using the magnetic module is effective and stable.

**Table 2. Immunoassay results using the magnetic modules**

| Antigen concentration µg/mL | Experiment 1 | Experiment 2 |
|---|---|---|
| 10 | 57168 | 51475 |
| 2.5 | 38346 | 38184 |
| 0.625 | 33193 | 28636 |
| 0.15625 | 18973 | 18213 |
| 0.03906 | 6196 | 5863 |
| 0.00977 | 3713 | 3613 |
| 0 | 2861 | 2815 |
| | | |

### Aspects of the Invention

According to a first aspect of the invention there is a magnetic module for reacting or labeling magnetic microcarriers, the module comprising one or more well sets, each well set comprising:a reaction well; one or more reaction wash wells comprising a first reaction wash well and a last reaction wash well, each containing a reaction wash buffer, wherein the first reaction wash well and the last reaction wash well are the same well when there is only one reaction wash well in the well set; a labeling well containing labeling molecules in a labeling buffer; and one or more labeling wash wells comprising a first labeling wash well and a last labeling wash well, each containing a labeling wash buffer, wherein the first labeling wash well and the last labeling wash well are the same well when there is only one labeling wash well in the well set; one or more magnetic units, wherein each magnetic unit: is configured to move into, out of, and between each of the wells in a well set; is configured to attract the magnetic microcarriers and move the magnetic microcarriers into, out of, and between each of the wells in the well set; and comprises an upper end and a lower end; and one or more magnetic unit pockets, wherein each magnetic unit pocket: is positioned between each magnetic unit and a well set; is configured to move into, out of, and between each of the well in the well set; is configured to at least partially cover the lower end of each magnetic unit when the magnetic unit moves or slides into the magnetic unit pocket; and comprises an interior surface and an exterior surface, wherein the magnetic microcarriers are coupled to one or more capture agents, wherein the one or more capture agents can bind to one or more analytes.

According to another aspect of the invention wherein the one or more capture agents are each independently selected from the group consisting of small molecules, polynucleotides, polypeptides, proteins, lipids, and polysaccharides, and wherein the one or more analytes are each independently selected from the group consisting of small molecules, polynucleotides, polypeptides, proteins, lipids, and polysaccharides.

According to another aspect of the invention wherein the one or more capture agents and the one or more analytes are each independently selected from the group consisting of polynucleotides; the one or more capture agents are each independently selected from the group consisting of antibodies and antibody fragments, and the one or more analytes are each independently selected from the group consisting of antigens; or the one or more capture agents are each independently selected from the group consisting of antigens, and the one or more analytes are each independently selected from the group consisting of antibodies.

According to another aspect of the invention wherein the labeling molecules comprise a label binding moiety coupled to a reporting moiety.

According to another aspect of the invention wherein the label binding moiety is streptavidin (SA) or anti-biotin antibody, and the reporting moiety is phycoerythrin (PE) or fluorescein isothiocyanate (FITC).

According to another aspect of the invention wherein each magnetic unit has a rod-like shape, and wherein each magnetic unit pocket has a closed-bottom cylindrical shape.

According to another aspect of the invention wherein the one or more magnetic unit pockets are configured to move or oscillate vertically relative to the one or more well sets.

According to another aspect of the invention wherein the number of the one or more magnetic units, the number of the one or more magnetic unit pockets, and the number of reaction wells are the same.

According to another aspect of the invention 1 wherein the one or more well sets form a multi-well plate, wherein each well set forms each row of the multi-well plate, and wherein each type of wells forms each column of the multi-well plate.

According to another aspect of the invention wherein the one or more magnetic units are positioned along a straight line above the multi-well plate and parallel to the column direction of the multi-well plate, and the one or more magnetic unit pockets are positioned along a straight line between the multi-well plate and the one or more magnetic units and parallel to the column direction of the multi-well plate.

According to another aspect of the invention wherein the reaction wells are positioned along a surface or form a two-dimensional arrangement, or wherein the labeling wells are positioned along a surface or form a two-dimensional arrangement, or both.

According to another aspect of the invention wherein each type of wells forms a multi-well plate, such that the one or more well sets comprise a plurality of multi-well plates.

According to another aspect of the invention wherein each type of wells is positioned along a line or forms a one-dimensional arrangement, the one or more magnetic units, the one or more magnetic unit pockets, and each type of wells are in the same one- or two-dimensional arrangement such that the one or more magnetic units can simultaneously move into and out of each type of wells, and the one or more magnetic unit pockets can simultaneously move into and out of each type of wells.

According to another aspect of the invention wherein the magnetic microcarriers comprise encoded microcarriers, spherical magnetic beads, or a combination thereof.

According to another aspect of the invention wherein the magnetic microcarriers comprise encoded microcarriers, and the encoded microcarriers each comprises: (a) a substantially transparent magnetic polymer layer having a first surface and a second surface, the first and the second surfaces being parallel to each other, wherein the substantially transparent magnetic polymer comprises a mixture of a substantially transparent polymer and a plurality of magnetic nanoparticles, and wherein the magnetic nanoparticles comprise iron(II,III) oxide or iron(III) oxide; (b) a substantially non-transparent layer, wherein the substantially non-transparent layer is affixed to the first surface of the substantially transparent magnetic polymer layer, and wherein an outline of the substantially non-transparent layer constitutes a two-dimensional shape representing an analog code; and (c) one or more capture agents for capturing one or more analytes, wherein the one or more capture agents are coupled to at least one of the first surface and the second surface of the substantially transparent magnetic polymer layer.

According to another aspect of the invention wherein the magnetic nanoparticles are superparamagnetic.

According to another aspect of the invention wherein the microcarrier is between about 5 µm and about 200 µm in diameter, or wherein the microcarrier is about 40 µm in diameter.

According to another aspect of the invention wherein the one or more analytes and the one or more capture agents are each independently selected from the group consisting of a DNA molecule, a DNA-analog-molecule, an RNA-molecule, an RNA-analog-molecule, a polynucleotide, a protein, an enzyme, a lipid, a phospholipid, a carbohydrate moiety, a polysaccharide, an antigen, a virus, a cell, an antibody, a small molecule, a bacterial cell, a cellular organelle, and an antibody fragment.

According to another aspect of the invention there is an assay system comprising the magnetic module of claim 1.

According to another aspect of the invention wherein the assay system further comprises a detection module configured to detect signal from the magnetic microcarriers.

According to another aspect of the invention wherein the detection module comprises one or more detection wells, and the one or more detection wells comprise the last labeling wash well.

According to another aspect of the invention wherein the detection module comprises one or more detection wells, and the one or more detection wells, the one or more magnetic units, and the one or more magnetic unit pockets have the same one- or two-dimensional arrangement such that the one or more magnetic units can simultaneously move into and out of the one or more detection wells, and the one or more magnetic unit pockets can simultaneously move into and out of the one or more detection wells.

According to another aspect of the invention, the essay system further comprising a PCR module configured to perform polymerase chain reaction (PCR).

According to another aspect of the invention the essay system further comprising a liquid handling module, wherein the liquid handling module is configured to transfer sample from the PCR module to the reaction well of the magnetic module.

According to another aspect of the invention wherein each well set further comprises a hybridization buffer well containing a hybridization buffer, and the liquid handling module is configured to transfer at least part of the hybridization buffer from the hybridization buffer well to the reaction well.

According to another aspect of the invention wherein each well set further comprises an incubation buffer well containing an incubation buffer, and the liquid handling module is configured to transfer at least part of the incubation buffer from the incubation buffer well to the reaction well.

According to another aspect of the invention there is desclosed a method of using the magnetic module, the method comprising:
(a) transferring one or more analytes into the reaction well, wherein the reaction well contains the magnetic microcarriers in a reaction buffer;
(b) moving one of the one or more magnetic unit pockets into the reaction well and moving or oscillating the magnetic unit pocket in the reaction well to mix the one or more analytes and the magnetic microcarriers;
(c) moving the magnetic unit into the reaction well and into the magnetic unit pocket to attract the magnetic microcarriers to the exterior surface of the magnetic unit pocket;
(d) moving the magnetic unit pocket and magnetic unit out of the reaction well and into the first reaction wash well to transfer the magnetic microcarriers from the reaction well to the first reaction wash well;
(e) moving the magnetic unit pocket and magnetic unit out of the last reaction wash well and into the labeling well to transfer the magnetic microcarriers from the last reaction wash well to the labeling well;
(f) moving the magnetic unit out of the labeling well while keeping the magnetic unit pocket in the labeling well, thereby releasing the magnetic microcarriers from the exterior surface of the magnetic unit pocket into the labeling buffer;
(g) moving the magnetic unit into the labeling well and into the magnetic unit pocket to attract the magnetic microcarriers to the exterior surface of the magnetic unit pocket; and
(h) moving the magnetic unit pocket and magnetic unit out of the labeling well and into the first labeling wash well to transfer the magnetic microcarriers from the labeling well to the first labeling wash well.

According to another aspect of the invention wherein steps (a) to (h) occur in the order listed.

According to another aspect of the invention further comprising, after step (d) and before step (e): moving the magnetic unit out of the first reaction wash well while keeping the magnetic unit pocket in the first reaction wash well, thereby releasing the magnetic microcarriers from the exterior surface of the magnetic unit pocket into the first reaction wash well; and moving or oscillating the magnetic unit pocket in the first reaction wash well to mix the magnetic microcarriers with the reaction wash buffer.

According to another aspect of the invention further comprising, after step (f) and before step (g), moving or oscillating the magnetic unit pocket in the labeling well to mix the magnetic microcarriers with the labeling buffer.

According to another aspect of the invention further comprising, after step (h): moving the magnetic unit out of the first labeling wash well while keeping the magnetic unit pocket in the first labeling wash well, thereby releasing the magnetic microcarriers from the exterior surface of the magnetic unit pocket into the first labeling wash well; and moving or oscillating the magnetic unit pocket in the first labeling wash well to mix the magnetic microcarriers with the labeling wash buffer.

According to another aspect of the invention further comprising, after step (h), moving the magnetic unit pocket and magnetic unit out of the last labeling wash well and into a detection well to transfer the magnetic microcarriers from the last reaction wash well to the detection well.

According to another aspect of the invention further comprising moving the magnetic unit out of the detection well while keeping the magnetic unit pocket in the detection well, thereby releasing the magnetic microcarriers from the exterior surface of the magnetic unit pocket into the detection well.

According to another aspect of the invention further comprising, after step (h), optically reading the magnetic microcarriers and detecting signal from labeling molecules coupled to the magnetic microcarriers.

According to another aspect of the invention there is disclosed a method of using the magnetic module, the method comprising:
(a) transferring one or more analytes into the reaction well, wherein the reaction well contains the magnetic microcarriers in a reaction buffer;
(b) moving one of the one or more magnetic unit pockets into the reaction well and moving or oscillating the magnetic unit pocket in the reaction well to mix the one or more analytes and the magnetic microcarriers;
(c) moving the magnetic unit into the reaction well and into the magnetic unit pocket to attract the magnetic microcarriers to the exterior surface of the magnetic unit pocket;
(d) moving the magnetic unit pocket and magnetic unit out of the reaction well and into the first reaction wash well to transfer the magnetic microcarriers from the reaction well to the first reaction wash well;
(e) moving the magnetic unit pocket and magnetic unit out of the last reaction wash well and into the antibody well to transfer the magnetic microcarriers from the last reaction wash well to the antibody well;
(f) moving the magnetic unit out of the antibody well while keeping the magnetic unit pocket in the labeling well, thereby releasing the magnetic microcarriers from the exterior surface of the magnetic unit pocket into the antibody buffer;
(g) moving the magnetic unit into the antibody well and into the magnetic unit pocket to attract the magnetic microcarriers to the exterior surface of the magnetic unit pocket;
(h) moving the magnetic unit pocket and magnetic unit out of the antibody well and into the first antibody wash well to transfer the magnetic microcarriers from the antibody well to the first antibody wash well;
(i) moving the magnetic unit pocket and magnetic unit out of the last antibody wash well and into the labeling well to transfer the magnetic microcarriers from the last antibody wash well to the labeling well;
(j) moving the magnetic unit out of the labeling well while keeping the magnetic unit pocket in the labeling well, thereby releasing the magnetic microcarriers from the exterior surface of the magnetic unit pocket into the labeling buffer;
(k) moving the magnetic unit into the labeling well and into the magnetic unit pocket to attract the magnetic microcarriers to the exterior surface of the magnetic unit pocket; and
(l) moving the magnetic unit pocket and magnetic unit out of the labeling well and into the first labeling wash well to transfer the magnetic microcarriers from the labeling well to the first labeling wash well.

According to another aspect of the invention wherein steps (a) to (1) occur in the order listed.

According to another aspect of the invention further comprising, after step (d) and before step (e): moving the magnetic unit out of the first reaction wash well while keeping the magnetic unit pocket in the first reaction wash well, thereby releasing the magnetic microcarriers from the exterior surface of the magnetic unit pocket into the first reaction wash well; and moving or oscillating the magnetic unit pocket in the first reaction wash well to mix the magnetic microcarriers with the reaction wash buffer.

According to another aspect of the invention further comprising, after step (f) and before step (g), moving or oscillating the magnetic unit pocket in the antibody well to mix the magnetic microcarriers with the labeling buffer.

According to another aspect of the invention further comprising, after step (h) and before step (i): moving the magnetic unit out of the first antibody wash well while keeping the magnetic unit pocket in the first antibody wash well, thereby releasing the magnetic microcarriers from the exterior surface of the magnetic unit pocket into the first antibody wash well; and moving or oscillating the magnetic unit pocket in the first antibody wash well to mix the magnetic microcarriers with the antibody wash buffer.

According to another aspect of the invention further comprising, after step (j) and before step (k), moving or oscillating the magnetic unit pocket in the labeling well to mix the magnetic microcarriers with the labeling buffer.

According to another aspect of the invention further comprising, after step (l): moving the magnetic unit out of the first labeling wash well while keeping the magnetic unit pocket in the first labeling wash well, thereby releasing the magnetic microcarriers from the exterior surface of the magnetic unit pocket into the first labeling wash well; and moving or oscillating the magnetic unit pocket in the first labeling wash well to mix the magnetic microcarriers with the labeling wash buffer.

According to another aspect of the invention further comprising, after step (l), moving the magnetic unit pocket and magnetic unit out of the last labeling wash well and into a detection well to transfer the magnetic microcarriers from the last reaction wash well to the detection well.

According to another aspect of the invention further comprising moving the magnetic unit out of the detection well while keeping the magnetic unit pocket in the detection well, thereby releasing the magnetic microcarriers from the exterior surface of the magnetic unit pocket into the detection well.

According to another aspect of the invention further comprising, after step (l), optically reading the magnetic microcarriers and detecting signal from labeling molecules coupled to the magnetic microcarriers.

According to another aspect of the invention there is disclosed a method of using an assay system comprising the magnetic module, the method comprising:
(a) transferring one or more analytes into the reaction well, wherein the reaction well contains the magnetic microcarriers in a reaction buffer;
(b) moving one of the one or more magnetic unit pockets into the reaction well and moving or oscillating the magnetic unit pocket in the reaction well to mix the one or more analytes and the magnetic microcarriers;
(c) moving the magnetic unit into the reaction well and into the magnetic unit pocket to attract the magnetic microcarriers to the exterior surface of the magnetic unit pocket;
(d) moving the magnetic unit pocket and magnetic unit out of the reaction well and into the first reaction wash well to transfer the magnetic microcarriers from the reaction well to the first reaction wash well;
(e) moving the magnetic unit pocket and magnetic unit out of the last reaction wash well and into the labeling well to transfer the magnetic microcarriers from the last reaction wash well to the labeling well;
(f) moving the magnetic unit out of the labeling well while keeping the magnetic unit pocket in the labeling well, thereby releasing the magnetic microcarriers from the exterior surface of the magnetic unit pocket into the labeling buffer;
(g) moving the magnetic unit into the labeling well and into the magnetic unit pocket to attract the magnetic microcarriers to the exterior surface of the magnetic unit pocket; and
(h) moving the magnetic unit pocket and magnetic unit out of the labeling well and into the first labeling wash well to transfer the magnetic microcarriers from the labeling well to the first labeling wash well.

According to another aspect of the invention there is disclosed a method of using an assay system comprising the magnetic module, the method comprising:
(a) transferring one or more analytes into the reaction well, wherein the reaction well contains the magnetic microcarriers in a reaction buffer;
(b) moving one of the one or more magnetic unit pockets into the reaction well and moving or oscillating the magnetic unit pocket in the reaction well to mix the one or more analytes and the magnetic microcarriers;
(c) moving the magnetic unit into the reaction well and into the magnetic unit pocket to attract the magnetic microcarriers to the exterior surface of the magnetic unit pocket;
(d) moving the magnetic unit pocket and magnetic unit out of the reaction well and into the first reaction wash well to transfer the magnetic microcarriers from the reaction well to the first reaction wash well;
(e) moving the magnetic unit pocket and magnetic unit out of the last reaction wash well and into the antibody well to transfer the magnetic microcarriers from the last reaction wash well to the antibody well;
(f) moving the magnetic unit out of the antibody well while keeping the magnetic unit pocket in the labeling well, thereby releasing the magnetic microcarriers from the exterior surface of the magnetic unit pocket into the antibody buffer;
(g) moving the magnetic unit into the antibody well and into the magnetic unit pocket to attract the magnetic microcarriers to the exterior surface of the magnetic unit pocket;
(h) moving the magnetic unit pocket and magnetic unit out of the antibody well and into the first antibody wash well to transfer the magnetic microcarriers from the antibody well to the first antibody wash well;
(i) moving the magnetic unit pocket and magnetic unit out of the last antibody wash well and into the labeling well to transfer the magnetic microcarriers from the last antibody wash well to the labeling well;
(j) moving the magnetic unit out of the labeling well while keeping the magnetic unit pocket in the labeling well, thereby releasing the magnetic microcarriers from the exterior surface of the magnetic unit pocket into the labeling buffer;
(k) moving the magnetic unit into the labeling well and into the magnetic unit pocket to attract the magnetic microcarriers to the exterior surface of the magnetic unit pocket; and
(l) moving the magnetic unit pocket and magnetic unit out of the labeling well and into the first labeling wash well to transfer the magnetic microcarriers from the labeling well to the first labeling wash well.

## Claims

1. A magnetic module for reacting or labeling magnetic microcarriers, the module comprising:
one or more well sets, each well set comprising:
a reaction well;
one or more reaction wash wells comprising a first reaction wash well and a last reaction wash well, each containing a reaction wash buffer, wherein the first reaction wash well and the last reaction wash well are the same well when there is only one reaction wash well in the well set;
a labeling well containing labeling molecules in a labeling buffer; and
one or more labeling wash wells comprising a first labeling wash well and a last labeling wash well, each containing a labeling wash buffer, wherein the first labeling wash well and the last labeling wash well are the same well when there is only one labeling wash well in the well set;
one or more magnetic units, wherein each magnetic unit:
is configured to move into, out of, and between each of the wells in a well set;
is configured to attract the magnetic microcarriers and move the magnetic microcarriers into, out of, and between each of the wells in the well set; and
comprises an upper end and a lower end; and
one or more magnetic unit pockets, wherein each magnetic unit pocket:
is positioned between each magnetic unit and a well set;
is configured to move into, out of, and between each of the well in the well set;
is configured to at least partially cover the lower end of each magnetic unit when the magnetic unit moves or slides into the magnetic unit pocket; and
comprises an interior surface and an exterior surface,
wherein the magnetic microcarriers are coupled to one or more capture agents, wherein the one or more capture agents can bind to one or more analytes.

2. The magnetic module of claim 1, wherein each well set further comprises:
an antibody well containing secondary antibodies in an antibody buffer, wherein the secondary antibodies can bind to the one or more analytes and are coupled to one or more secondary binding moieties; and
one or more antibody wash wells, comprising a first antibody wash well and a last antibody wash well, each containing an antibody wash buffer, wherein the first antibody wash well and the last antibody wash well are the same well when there is only one antibody wash well in the well set.

3. The magnetic module of claim 2, wherein the reaction well, one or more reaction wash wells, antibody well, one or more antibody wash wells, labeling well, and one or more labeling wash wells are positioned along a straight or curved line in such order.

4. The magnetic module of claim 1, wherein the reaction well, one or more reaction wash wells, labeling well, and one or more labeling wash wells are positioned along a straight or curved line in such order.

5. The magnetic module of any one of claims 1, wherein the one or more capture agents are each independently selected from the group consisting of small molecules, polynucleotides, polypeptides, proteins, lipids, and polysaccharides, and wherein the one or more analytes are each independently selected from the group consisting of small molecules, polynucleotides, polypeptides, proteins, lipids, and polysaccharides.

6. The magnetic module of any one of claims 1, wherein:
the one or more capture agents and the one or more analytes are each independently selected from the group consisting of polynucleotides;
the one or more capture agents are each independently selected from the group consisting of antibodies and antibody fragments, and the one or more analytes are each independently selected from the group consisting of antigens; or
the one or more capture agents are each independently selected from the group consisting of antigens, and the one or more analytes are each independently selected from the group consisting of antibodies.

7. The magnetic module of any one of claims 1, wherein the labeling molecules comprise a label binding moiety coupled to a reporting moiety.

8. The magnetic module of claim 7, wherein the label binding moiety is streptavidin (SA) or anti-biotin antibody, and the reporting moiety is phycoerythrin (PE) or fluorescein isothiocyanate (FITC).

9. The magnetic module of any one of claims 1, wherein each magnetic unit has a rod-like shape, and wherein each magnetic unit pocket has a closed-bottom cylindrical shape.

10. The magnetic module of claim 1, wherein the one or more magnetic unit pockets are configured to move or oscillate vertically relative to the one or more well sets.

11. The magnetic module of claim 1, wherein the number of the one or more magnetic units, the number of the one or more magnetic unit pockets, and the number of reaction wells are the same.

12. The magnetic module of claim 1, wherein the one or more well sets form a multi-well plate, wherein each well set forms each row of the multi-well plate, and wherein each type of wells forms each column of the multi-well plate.

13. The magnetic module of claim 12, wherein the one or more magnetic units are positioned along a straight line above the multi-well plate and parallel to the column direction of the multi-well plate, and the one or more magnetic unit pockets are positioned along a straight line between the multi-well plate and the one or more magnetic units and parallel to the column direction of the multi-well plate.

14. The magnetic module of claim 1, wherein the reaction wells are positioned along a surface or form a two-dimensional arrangement, or wherein the labeling wells are positioned along a surface or form a two-dimensional arrangement, or both.

15. The magnetic module of claim 1, wherein each type of wells forms a multi-well plate, such that the one or more well sets comprise a plurality of multi-well plates.

16. The magnetic module of claim 1, wherein each type of wells is positioned along a line or forms a one-dimensional arrangement, the one or more magnetic units, the one or more magnetic unit pockets, and each type of wells are in the same one- or two-dimensional arrangement such that the one or more magnetic units can simultaneously move into and out of each type of wells, and the one or more magnetic unit pockets can simultaneously move into and out of each type of wells.

17. The magnetic module of any one of claims 1, wherein the magnetic microcarriers comprise encoded microcarriers, spherical magnetic beads, or a combination thereof.

18. The magnetic module of claim 1, wherein the magnetic microcarriers comprise encoded microcarriers, and the encoded microcarriers each comprises:
(a) a substantially transparent magnetic polymer layer having a first surface and a second surface, the first and the second surfaces being parallel to each other, wherein the substantially transparent magnetic polymer comprises a mixture of a substantially transparent polymer and a plurality of magnetic nanoparticles, and wherein the magnetic nanoparticles comprise iron(II,III) oxide or iron(III) oxide;
(b) a substantially non-transparent layer, wherein the substantially non-transparent layer is affixed to the first surface of the substantially transparent magnetic polymer layer, and wherein an outline of the substantially non-transparent layer constitutes a two-dimensional shape representing an analog code; and
(c) one or more capture agents for capturing one or more analytes, wherein the one or more capture agents are coupled to at least one of the first surface and the second surface of the substantially transparent magnetic polymer layer.

19. The magnetic module of claim 18, wherein the magnetic nanoparticles are superparamagnetic.

20. The magnetic module of any one of claims 18, wherein the microcarrier is between about 5 µm and about 200 µm in diameter, or wherein the microcarrier is about 40 µm in diameter.

21. The magnetic module of any one of claims 18, wherein the one or more analytes and the one or more capture agents are each independently selected from the group consisting of a DNA molecule, a DNA-analog-molecule, an RNA-molecule, an RNA-analog-molecule, a polynucleotide, a protein, an enzyme, a lipid, a phospholipid, a carbohydrate moiety, a polysaccharide, an antigen, a virus, a cell, an antibody, a small molecule, a bacterial cell, a cellular organelle, and an antibody fragment.

22. An assay system comprising the magnetic module of claim 1.

23. The assay system of claim 22, wherein the system further comprises a detection module configured to detect signal from the magnetic microcarriers.

24. The assay system of claim 23, wherein the detection module comprises one or more detection wells, and the one or more detection wells comprise the last labeling wash well.

25. The assay system of claim 23, wherein the detection module comprises one or more detection wells, and the one or more detection wells, the one or more magnetic units, and the one or more magnetic unit pockets have the same one- or two-dimensional arrangement such that the one or more magnetic units can simultaneously move into and out of the one or more detection wells, and the one or more magnetic unit pockets can simultaneously move into and out of the one or more detection wells.

26. The assay system of claim 22, further comprising a PCR module configured to perform polymerase chain reaction (PCR).

27. The assay system of claim 22, further comprising a liquid handling module, wherein the liquid handling module is configured to transfer sample from the PCR module to the reaction well of the magnetic module.

28. The assay system of claim 27, wherein each well set further comprises a hybridization buffer well containing a hybridization buffer, and the liquid handling module is configured to transfer at least part of the hybridization buffer from the hybridization buffer well to the reaction well.

29. The assay system of claim 27, wherein each well set further comprises an incubation buffer well containing an incubation buffer, and the liquid handling module is configured to transfer at least part of the incubation buffer from the incubation buffer well to the reaction well.

30. A method of using the magnetic module of claim 1, the method comprising:
(i) transferring one or more analytes into the reaction well, wherein the reaction well contains the magnetic microcarriers in a reaction buffer;
(j) moving one of the one or more magnetic unit pockets into the reaction well and moving or oscillating the magnetic unit pocket in the reaction well to mix the one or more analytes and the magnetic microcarriers;
(k) moving the magnetic unit into the reaction well and into the magnetic unit pocket to attract the magnetic microcarriers to the exterior surface of the magnetic unit pocket;
(l) moving the magnetic unit pocket and magnetic unit out of the reaction well and into the first reaction wash well to transfer the magnetic microcarriers from the reaction well to the first reaction wash well;
(m)moving the magnetic unit pocket and magnetic unit out of the last reaction wash well and into the labeling well to transfer the magnetic microcarriers from the last reaction wash well to the labeling well;
(n) moving the magnetic unit out of the labeling well while keeping the magnetic unit pocket in the labeling well, thereby releasing the magnetic microcarriers from the exterior surface of the magnetic unit pocket into the labeling buffer;
(o) moving the magnetic unit into the labeling well and into the magnetic unit pocket to attract the magnetic microcarriers to the exterior surface of the magnetic unit pocket; and
(p) moving the magnetic unit pocket and magnetic unit out of the labeling well and into the first labeling wash well to transfer the magnetic microcarriers from the labeling well to the first labeling wash well.

31. The method of claim 30, wherein steps (a) to (h) occur in the order listed.

32. The method of claim 30, further comprising, after step (d) and before step (e):
moving the magnetic unit out of the first reaction wash well while keeping the magnetic unit pocket in the first reaction wash well, thereby releasing the magnetic microcarriers from the exterior surface of the magnetic unit pocket into the first reaction wash well; and
moving or oscillating the magnetic unit pocket in the first reaction wash well to mix the magnetic microcarriers with the reaction wash buffer.

33. The method of claim 30, further comprising, after step (f) and before step (g), moving or oscillating the magnetic unit pocket in the labeling well to mix the magnetic microcarriers with the labeling buffer.

34. The method of claim 30, further comprising, after step (h):
moving the magnetic unit out of the first labeling wash well while keeping the magnetic unit pocket in the first labeling wash well, thereby releasing the magnetic microcarriers from the exterior surface of the magnetic unit pocket into the first labeling wash well; and
moving or oscillating the magnetic unit pocket in the first labeling wash well to mix the magnetic microcarriers with the labeling wash buffer.

35. The method of claim 30, further comprising, after step (h), moving the magnetic unit pocket and magnetic unit out of the last labeling wash well and into a detection well to transfer the magnetic microcarriers from the last reaction wash well to the detection well.

36. The method of claim 35, further comprising moving the magnetic unit out of the detection well while keeping the magnetic unit pocket in the detection well, thereby releasing the magnetic microcarriers from the exterior surface of the magnetic unit pocket into the detection well.

37. The method of claim 30, further comprising, after step (h), optically reading the magnetic microcarriers and detecting signal from labeling molecules coupled to the magnetic microcarriers.

38. A method of using the magnetic module of claim 2, the method comprising:
(m)transferring one or more analytes into the reaction well, wherein the reaction well contains the magnetic microcarriers in a reaction buffer;
(n) moving one of the one or more magnetic unit pockets into the reaction well and moving or oscillating the magnetic unit pocket in the reaction well to mix the one or more analytes and the magnetic microcarriers;
(o) moving the magnetic unit into the reaction well and into the magnetic unit pocket to attract the magnetic microcarriers to the exterior surface of the magnetic unit pocket;
(p) moving the magnetic unit pocket and magnetic unit out of the reaction well and into the first reaction wash well to transfer the magnetic microcarriers from the reaction well to the first reaction wash well;
(q) moving the magnetic unit pocket and magnetic unit out of the last reaction wash well and into the antibody well to transfer the magnetic microcarriers from the last reaction wash well to the antibody well;
(r) moving the magnetic unit out of the antibody well while keeping the magnetic unit pocket in the labeling well, thereby releasing the magnetic microcarriers from the exterior surface of the magnetic unit pocket into the antibody buffer;
(s) moving the magnetic unit into the antibody well and into the magnetic unit pocket to attract the magnetic microcarriers to the exterior surface of the magnetic unit pocket;
(t) moving the magnetic unit pocket and magnetic unit out of the antibody well and into the first antibody wash well to transfer the magnetic microcarriers from the antibody well to the first antibody wash well;
(u) moving the magnetic unit pocket and magnetic unit out of the last antibody wash well and into the labeling well to transfer the magnetic microcarriers from the last antibody wash well to the labeling well;
(v) moving the magnetic unit out of the labeling well while keeping the magnetic unit pocket in the labeling well, thereby releasing the magnetic microcarriers from the exterior surface of the magnetic unit pocket into the labeling buffer;
(w) moving the magnetic unit into the labeling well and into the magnetic unit pocket to attract the magnetic microcarriers to the exterior surface of the magnetic unit pocket; and
(x) moving the magnetic unit pocket and magnetic unit out of the labeling well and into the first labeling wash well to transfer the magnetic microcarriers from the labeling well to the first labeling wash well.

39. The method of claim 38, wherein steps (a) to (l) occur in the order listed.

40. The method of claim 38, further comprising, after step (d) and before step (e):
moving the magnetic unit out of the first reaction wash well while keeping the magnetic unit pocket in the first reaction wash well, thereby releasing the magnetic microcarriers from the exterior surface of the magnetic unit pocket into the first reaction wash well; and
moving or oscillating the magnetic unit pocket in the first reaction wash well to mix the magnetic microcarriers with the reaction wash buffer.

41. The method of claim 38, further comprising, after step (f) and before step (g), moving or oscillating the magnetic unit pocket in the antibody well to mix the magnetic microcarriers with the labeling buffer.

42. The method of claim 38, further comprising, after step (h) and before step (i):
moving the magnetic unit out of the first antibody wash well while keeping the magnetic unit pocket in the first antibody wash well, thereby releasing the magnetic microcarriers from the exterior surface of the magnetic unit pocket into the first antibody wash well; and
moving or oscillating the magnetic unit pocket in the first antibody wash well to mix the magnetic microcarriers with the antibody wash buffer.

43. The method of claim 38, further comprising, after step (j) and before step (k), moving or oscillating the magnetic unit pocket in the labeling well to mix the magnetic microcarriers with the labeling buffer.

44. The method of claim 38, further comprising, after step (l):
moving the magnetic unit out of the first labeling wash well while keeping the magnetic unit pocket in the first labeling wash well, thereby releasing the magnetic microcarriers from the exterior surface of the magnetic unit pocket into the first labeling wash well; and
moving or oscillating the magnetic unit pocket in the first labeling wash well to mix the magnetic microcarriers with the labeling wash buffer.

45. The method of claim 38, further comprising, after step (1), moving the magnetic unit pocket and magnetic unit out of the last labeling wash well and into a detection well to transfer the magnetic microcarriers from the last reaction wash well to the detection well.

46. The method of claim 45, further comprising moving the magnetic unit out of the detection well while keeping the magnetic unit pocket in the detection well, thereby releasing the magnetic microcarriers from the exterior surface of the magnetic unit pocket into the detection well.

47. The method of claim 38, further comprising, after step (l), optically reading the magnetic microcarriers and detecting signal from labeling molecules coupled to the magnetic microcarriers.

48. A method of using an assay system comprising the magnetic module of claim 1, the method comprising:
(i) transferring one or more analytes into the reaction well, wherein the reaction well contains the magnetic microcarriers in a reaction buffer;
(j) moving one of the one or more magnetic unit pockets into the reaction well and moving or oscillating the magnetic unit pocket in the reaction well to mix the one or more analytes and the magnetic microcarriers;
(k) moving the magnetic unit into the reaction well and into the magnetic unit pocket to attract the magnetic microcarriers to the exterior surface of the magnetic unit pocket;
(l) moving the magnetic unit pocket and magnetic unit out of the reaction well and into the first reaction wash well to transfer the magnetic microcarriers from the reaction well to the first reaction wash well;
(m)moving the magnetic unit pocket and magnetic unit out of the last reaction wash well and into the labeling well to transfer the magnetic microcarriers from the last reaction wash well to the labeling well;
(n) moving the magnetic unit out of the labeling well while keeping the magnetic unit pocket in the labeling well, thereby releasing the magnetic microcarriers from the exterior surface of the magnetic unit pocket into the labeling buffer;
(o) moving the magnetic unit into the labeling well and into the magnetic unit pocket to attract the magnetic microcarriers to the exterior surface of the magnetic unit pocket; and
(p) moving the magnetic unit pocket and magnetic unit out of the labeling well and into the first labeling wash well to transfer the magnetic microcarriers from the labeling well to the first labeling wash well.

49. A method of using an assay system comprising the magnetic module of claim 2, the method comprising:
(m)transferring one or more analytes into the reaction well, wherein the reaction well contains the magnetic microcarriers in a reaction buffer;
(n) moving one of the one or more magnetic unit pockets into the reaction well and moving or oscillating the magnetic unit pocket in the reaction well to mix the one or more analytes and the magnetic microcarriers;
(o) moving the magnetic unit into the reaction well and into the magnetic unit pocket to attract the magnetic microcarriers to the exterior surface of the magnetic unit pocket;
(p) moving the magnetic unit pocket and magnetic unit out of the reaction well and into the first reaction wash well to transfer the magnetic microcarriers from the reaction well to the first reaction wash well;
(q) moving the magnetic unit pocket and magnetic unit out of the last reaction wash well and into the antibody well to transfer the magnetic microcarriers from the last reaction wash well to the antibody well;
(r) moving the magnetic unit out of the antibody well while keeping the magnetic unit pocket in the labeling well, thereby releasing the magnetic microcarriers from the exterior surface of the magnetic unit pocket into the antibody buffer;
(s) moving the magnetic unit into the antibody well and into the magnetic unit pocket to attract the magnetic microcarriers to the exterior surface of the magnetic unit pocket;
(t) moving the magnetic unit pocket and magnetic unit out of the antibody well and into the first antibody wash well to transfer the magnetic microcarriers from the antibody well to the first antibody wash well;
(u) moving the magnetic unit pocket and magnetic unit out of the last antibody wash well and into the labeling well to transfer the magnetic microcarriers from the last antibody wash well to the labeling well;
(v) moving the magnetic unit out of the labeling well while keeping the magnetic unit pocket in the labeling well, thereby releasing the magnetic microcarriers from the exterior surface of the magnetic unit pocket into the labeling buffer;
(w) moving the magnetic unit into the labeling well and into the magnetic unit pocket to attract the magnetic microcarriers to the exterior surface of the magnetic unit pocket; and
(x) moving the magnetic unit pocket and magnetic unit out of the labeling well and into the first labeling wash well to transfer the magnetic microcarriers from the labeling well to the first labeling wash well.
